# EUROPEAN PATENT APPLICATION

(11) **EP 2 287 146 A1**
(43) Date of publication of application: **23.02.2011**
(21) Application number: 09742183.8
(22) Date of filing: 05.05.2009
(51) Int. Cl.: C07C 59/42, C07C 57/02, C07C 69/732

(54) **METHOD FOR PREPARING OREGANIC ACID**

(30) Priority: 06.05.2008 ES 200801304
(71) Applicant: Consejo Superior de Investigaciones Cientificas, 28006 Madrid (ES)
(72) Inventor: NOHEDA MARÍN, Pedro, E-28006 Madrid (ES); LOZANO GORDILLO, Luis Miguel, E-28006 Madrid (ES); MAROTO QUINTANA, Sergio, E-28006 Madrid (ES)
(74) Representative: ABG Patentes, S.L.
(86) International application number: PCT/ES2009/070138
(87) International publication number: WO 2009/135977

(57) **Abstract**

The present invention is aimed at a process for obtaining oreganic acid and derivatives thereof, to the intermediate compounds of this synthesis and to the use of these compounds in the preparation of oreganic acid and derivatives thereof.

## Description

### Field of the Invention

The present invention relates to a new process for the synthesis of oreganic acid and derivatives thereof, to the intermediate compounds of the synthesis and to the use of these compounds in the preparation of oreganic acid and their derivatives.

### State of the Art

The compound (*Z*)-3,4-dicarboxy-20-sulfooxy-3-eicosenoic acid, also known as oreganic acid, of formula **1*-Z*** 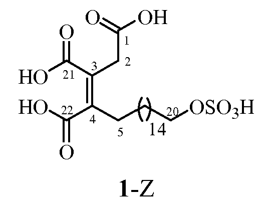 is a natural product belonging to the family of alkyl citrates and having a Ras FTase inhibitory activity. The inhibition of the farnesylation process of Ras proteins is considered as an effective mechanism for controlling the growth of tumors promoted by mutated Ras proteins, since said inhibition prevents mutated Ras proteins from being introduced in the cell membrane and being activated, not being able to carry out their biological activity. For a study of the biological activity of oreganic acid see: Silverman, K. C.; Jayasuriya, H.; Cascales, C.; Vilella, D.; Bills, G. F.; Jenkins, R. G.; Singh, S. B.; Lingham, R. B. Biochem. Biophis. Res. Commun. 1997, 232, 478-481.

Oreganic acid was isolated by Dr. Jayasuriya's group in the year 1996 from an unidentified endophytic fungus (MF 6046), isolated from living leaves of *Berberis oregana* (Berberidaceae) leaves in Lord Ellis Summit, Humboldt Co. California, USA. For the purpose of elucidating the structure of oreganic acid, the trimethylated ester derivative and their corresponding desulfated derivative were prepared.

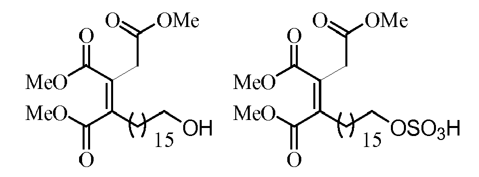

However, when elucidating their structure, E isomerism was incorrectly assigned to it (see Jayasuriya, H.; Bills, G. F.; Cascales, C.; Zink, D. L.; Goetz, M. A.; Jenkins, R. G.; Silverman, K. C.; Lingham, R. B.; Singh, S. B. Bioorg. Med. Chem. Lett. 1996, 6, 2081-2084)

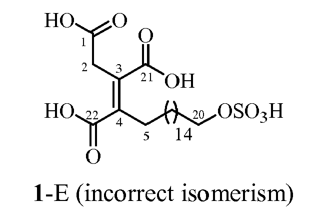

Subsequently, Dr. Gibbs' group represented oreganic acid with a Z (correct) geometry in the tetrasubstituted double bond between the C3-C4 positions (Gibbs, R. A.; Zahn, T. J.; Sebolt-Leopold, J. S Curr. Med. Chem. 2001, 8, 1437-1465). Nevertheless, data justifying that the configurational assignment was correct were not provided.

No total synthesis of oreganic acid has been described up until now. In this context, it would be convenient to provide a process for obtaining oreganic acid, which preferably meets the following characteristics:
- using a small number of protecting groups;
- feasible and inexpensive commercial starting substrates;
- a reasonable number of steps;
- non-sophisticated experimental processes; or
- a good final yield.

### Brief Description of the Invention

The authors of the invention have prepared the first total synthesis described to date of oreganic acid and derivatives thereof. Said synthesis uses simple starting substrates, involves a reasonable number of steps, needs few protecting groups and does not comprises complex transformations. Therefore, it allows obtaining oreganic acid with a good yield, and following conditions which would enable the synthesis at a large scale and in a short time.

Therefore, a first aspect of the invention relates to a process for obtaining a compound of formula (VIII), an intermediate of oreganic acid and their derivatives, which already comprises the final backbone of the molecule, which comprises subjecting a compound of formula (VI) or (VII) to a hydrogenation reaction; or deprotecting the trialkylsilyl group of a compound of formula (XVI).

Additional aspects of the invention are said compounds of formula (VI), (VII) and (XVI), their stereoisomers, or mixtures thereof, intermediates of the synthesis, and processes for obtaining them.

An additional aspect of the present invention relates to a process for obtaining compounds of formula (I), their stereoisomers, or mixtures thereof, from compounds of formula (VI), (VII) or (XVI), their stereoisomers, or mixtures thereof through compounds of formula (VIII), their stereoisomers, or mixtures thereof.

Additional aspects of the invention relate to compounds of formula (I), (IV), (V), (VI), (VII), (VIII), (IX), (XV), (XVI) and (XVIII) defined herein.

Another aspect of the invention corresponds to the use of at least one compound selected from the compounds of formula (II), (III), (IV), (V), (VI), (VII), (VIII), (X), (XI), (XII), (XIII), (XIV), (XV), (XVI), (XVIII) and (XXIV), their stereoisomers, or mixtures thereof, for the synthesis of compounds of formula (I), their stereoisomers, or mixtures thereof.

### Detailed Description of the Invention

### Definitions

For the purpose of facilitating the understanding of the present invention, the meanings of several terms and expressions as they are used in the context of the invention are herein included below.

"Alkyl" refers to a radical with a hydrocarbon chain which consists of carbon and hydrogen atoms, which does not contain unsaturations and which is attached to the rest of the molecule by means of a single bond. The number of carbon atoms of the alkyl group is specified in each case. For example, when "C₁-C₄ alkyl" is indicated, it refers to an alkyl group of one, two, three of four carbon atoms, i.e., methyl, ethyl, propyl, isopropyl, n-butyl, sec-butyl or tert-butyl.

"Aryl" refers to an aromatic substituent, preferably C₆-C₁₈, more preferably C₆-C₁₄, more preferably C₆-C₁₀, which comprises a simple aromatic ring or multiple fused rings in which at least one of them is aromatic. Preferred aryl groups are phenyl or naphthyl, preferably phenyl.

"Halogen" means -F, -Cl, -Br or -I.

"Trialkylsilyl" is understood as a radical of formula -Si(R')(R")R"', wherein each of R', R" and R"' are independently selected from among a phenyl group and a C₁-C₆ alkyl group. Non-limiting examples of trialkylsilyl groups can be trimethylsilyl, triethylsilyl, tri-*iso*-propylsilyl; dimethylisopropylsilyl, diethylisopropylsilyl, dimethylthexylsilyl, *tert*-butyldimethylsilyl, *tert*-butyldiphenylsilyl.

"Bn" means benzyl (-(CH₂)-phenyl).

"Leaving group" is an atom or group of atoms activating the carbon to which they are attached against a nucleophilic reagent, such that said nucleophile, or part of said nucleophile, is attached to the molecule and the leaving group is detached therefrom. Leaving groups are known by the person skilled in the art, for example, OTs (tosyl), OMs (mesyl) or halogen.

The references of the present document to substituted groups in the compounds of the present invention refer to the specified moiety which can be substituted in one, two or three available positions with one, two, three suitable groups, which are independently selected from the group consisting of cyano; alkanoyl, such as a C₁-C₆ alkanoyl group, such as acyl and the like; carboxamido (-(C=O)NH₂); trialkylsilyl. As a non-limiting example, "substituted alkyl" includes groups such as cyanoethyl, acetylmethyl, carboxamidomethyl (-CH₂CONH₂), 2-trimethylsilylethyl.

Throughout the present invention, when positions of the different compounds are mentioned, the numbering followed is:

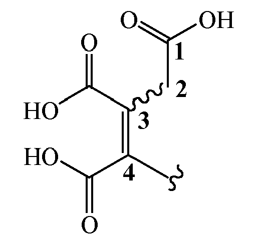

The chain attached to the carbon of position 4 will be referred to as "side chain". As will be discussed below, all the compounds of the present invention containing a double bond between the C3-C4 positions can have said double bond with a *Z* configuration, with an *E* configuration or can be found as mixtures of *Z*/*E* isomers. The present invention includes said stereoisomers or mixtures thereof, all of them being useful in the synthesis of compounds of formula (I). One of the substituents of said double bond attached by means of a "wavy" bond to highlight this possibility.

Unless otherwise indicated, the compounds of the invention also refer to those including compounds which differ only in the presence of one or more isotopically enriched atoms. For example, the compounds having the present structures, with the exception of the substitution of a hydrogen with a deuterium or with tritium, or the substitution of a carbon with a ¹³C- or ¹⁴C-enriched carbon, are within the scope of this invention.

The reaction products obtained can be purified, if desired, by means of conventional methods, such as crystallization, chromatography and trituration.

### Synthesis

According to a first aspect, the present invention relates to a process for obtaining a compound of formula (VIII), its stereoisomers or mixtures thereof 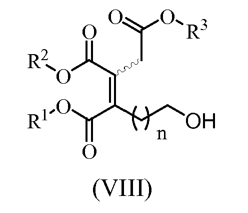 wherein
R¹, R² and R³ are independently selected from substituted or unsubstituted C₁-C₂₀ alkyl; and
n is an integer between 2 and 20;
characterized in that it comprises at least one of the following steps (a), (b) or (c)
(a) subjecting a compound of formula (VI), its stereoisomers or mixtures thereof, to a hydrogenation reaction 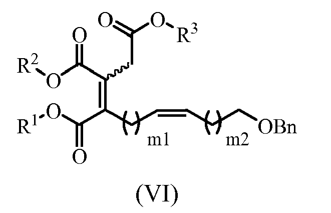 wherein
   R¹, R² and R³ are as defined above;
   m1 is an integer which is selected from 1, 2, 3, 4 and 5; and
   m2 is an integer between 0 and 13;
(b) subjecting a compound of formula (VII), its stereoisomers or mixtures thereof, to a hydrogenation reaction 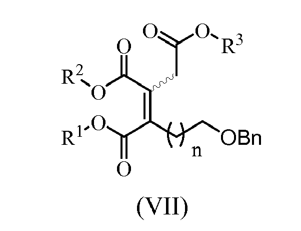 wherein
   n, R¹, R² and R³ are as defined above;
   or
(c) removing the trialkylsilyl group of a compound of formula (XVI), its stereoisomers or mixtures thereof 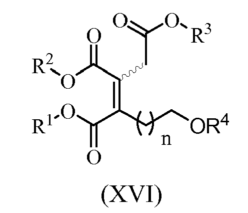 wherein
   n, R¹, R² and R³ are as defined above; and
   R⁴ is a trialkylsilyl group.

In a preferred embodiment, a metal catalyst is added on a solution of a trimester of formula (VI) or (VII) in a suitable solvent, such as 1,4-dioxane for example. Preferably, the solvent is degassed by means of passing a stream of an inert gas. The suspension thus prepared is stirred under a hydrogen atmosphere for a time between 10 and 210 minutes, preferably between 20 and 150 minutes, more preferably between 25 and 80 minutes.

The conditions under which heterogeneous hydrogenation reactions are performed are known for the person skilled in the art. For a review on heterogeneous hydrogenation reactions see, for example: Nishimura, S. Heterogenous Catalytic Hydrogenation for Organic Synthesis; John Wiley & Sons, 2001. Various metal catalysts are used, in which the metal is selected from platinum, palladium, rhodium or ruthenium. Some commercial catalysts of the hydrogenation reaction useful for the purposes of the present invention are, for example, Ni(Ra), Pd/C, Rh(Al₂O₃). Among them, the preferred catalyst is Pd/C.

As can be seen, in the case of the compounds of formula (VI), it is thus possible to reduce the double bond of the side chain rather than the conjugated double bond existing between the C3-C4 carbons, and release the end hydroxyl group of the side chain. In the case of the compounds of formula (VII) it is enough to release the end hydroxyl of the side chain. Alternatively, it is possible to only reduce the double bond of the side chain to a compound of formula (VI) to obtain a compound of formula (VII), which can be reduced in the same reaction medium or after isolation, to a compound of formula (VIII). In other words, the reduction of the compounds of formula (VI) to the compounds of formula (VIII) can be performed in a single step, or in two steps through a compound of formula (VII).

For example, conditions under which the compound of formula (VI) is relatively diluted in the reaction medium (0.02-1.5 M), preferably 0.07-1.5 M, and Pd/C of a concentration between 3 and 7%, preferably 5%, favor directly obtaining an alcohol of formula (VIII) as a single product. Preferred embodiments for directly obtaining compounds of formula (VIII) are also those wherein between 0.01-0.1 equivalents, preferably 0.04-0.06 equivalents, of Pd/C are added. According to another preferred embodiment, the solvent is 1,4-dioxane. The reaction time is preferably between 50 and 70 minutes.

Preferred conditions for obtaining a compound of formula (VII) from compounds of formula (VI) are those in which the concentration of the compound of formula (VI) in the reaction medium and the concentration of Pd/C is slightly higher. Thus, for example, according to a preferred embodiment the concentration of the compound of formula (VI) in the reaction medium is greater than 0.15 M, preferably of 0.16-0.25 M), more preferably about 0.2M, and Pd/C of a concentration between 8 and 12%, preferably 10%, is used. Preferred embodiments for obtaining compounds of formula (VII) are also those wherein between 0.01-0.1 equivalents, preferably 0.04-0.06 equivalents, of Pd/C are added. According to another preferred embodiment, the solvent is 1,4-dioxane. The reaction time is preferably between 10 and 50 minutes, preferably between 20 and 40 minutes, more preferably about 30 minutes.

As can be observed, option (c) comprises the removal of the trialkylsilyl group, i.e., the transformation of R⁴ into a hydrogen, of a compound of formula (XVI). Said transformation is normally understood as a deprotection and can be performed under different conditions (see for example, Kocienski, P. J. Protecting Groups; Thieme: Stuttgart, 2000. pp.: 187-230). According to a preferred embodiment, the trialkylsilyl group is removed from a compound of formula (XVI) to give rise to a compound of formula (VIII) in diluted acidic medium, such as 1% HCl, for example

According to another aspect, the invention relates to compounds of (VI), (VII), or (XVI) as defined above, immediate precursors of the compounds of formula (VIII). According to a preferred embodiment, in the compounds of formula (VII) or (XVI) n is an integer between 8 and 20, preferably between 12 and 17, more preferably between 13 and 16, more preferably n is 15. According to a preferred embodiment, in the compounds of formula (VI) m1 is an integer which is selected from 2, 3 or 4, preferably m1 is 3. According to a preferred embodiment, m2 is an integer which is selected from among 5-12, preferably 8-12, more preferably m2 is 10.

Said groups R¹, R² and R³ act as protecting groups for the acid groups of the compounds of formula (I) and, as seen below, they are removed in the last steps of the synthesis. Therefore, any substituted or unsubstituted C₁-C₂₀ alkyl protecting group, orthogonal to the rest of the protecting groups used in the synthesis (for example, benzyl and trialkylsilyl) is useful for the purposes of the present invention. Preferably, each of R¹, R² and R³ is independently selected from a C₁-C₄, preferably C₁₋₃, alkyl group more preferably, R¹, R² and R³ are methyl groups.

Another aspect of the present invention relates to a process for the synthesis of a compound of formula (VI). Said compound of formula (VI), its stereoisomers or mixtures thereof, can be prepared by reacting a compound of formula (V) with a phosphonate of formula (XIII) in the presence of a base: 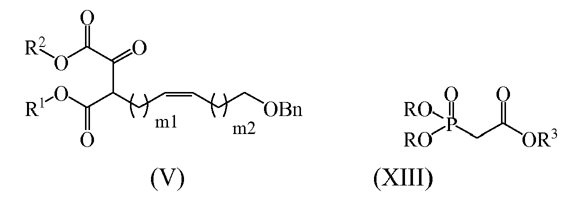 wherein
R¹ is selected from a substituted or unsubstituted C₁-C₂₀ alkyl; and
R is selected from C₁-C₄ alkyl substituted or unsubstituted with one or more halogen atoms, benzyl, phenyl and tolyl.

Reactions of this type are known in the state of the art and allow a certain variability in their conditions (for example, see Wittig see: Maryanoff, B. E.; Reizt, A. B. Chem. Rev. 1989, 89, 863-927). The phosphonate is preferably in an excess with respect to the compound of formula (V), more preferably in a proportion between 1.5 to 3 equivalents, still more preferably in a proportion of 2.2 equivalents. As has been mentioned, this transformation furthermore needs the presence of a base, such as lithium bases for example, such as *n-*BuLi, *t-*BuLi, *s-*BuLi, LDA, LiHMDS for example; sodium bases, such as NaH, NaHMDS for example; or potassium bases, such as KHMDS, *t*-BuOK for example. The base is preferably NaH. The amount of base depends on the amount of phosphonate used. Thus, the base is preferably in an excess with respect to the compound of formula (V), more preferably in a proportion between 1.5-3. In a preferred embodiment, the reaction is performed using THF at room temperature as a solvent.

Depending on the reaction conditions and the specific substituents, the reaction leads to mixtures of the C3-C4 geometric isomers of the compound (VI) in a different ratio. Both isomers are within the scope of the present invention and allow the synthesis of oreganic acid and its derivatives, as discussed below.

A compound of formula (V) can be prepared by reacting a compound of formula (IV) with an oxalate of formula (XII) in the presence of a base: 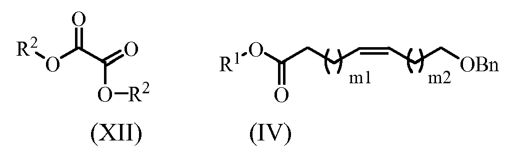 wherein
R¹ and R² are independently selected from a substituted or unsubstituted C₁-C₂₀ alkyl;
m1 is an integer which is selected from 0, 1, 2, 3, 4 and 5; and
m2 is an integer between 0 and 13.

As has been mentioned, this transformation furthermore needs the presence of a base, such as lithium bases for example, such as *n-*BuLi, *t-*BuLi, *s-*BuLi, LDA, LiHMDS for example; sodium bases, such as NaH, NaHMDS for example; or potassium bases, such as KHMDS, *t*-BuOK for example. The base is preferably NaH.

According to a preferred embodiment, the reaction is suitably performed in a mixture of MeOH/THF, using a temperature of about 70°C.

A compound of formula (IV) can be prepared by reacting a compound (III) with a compound of formula (XI): 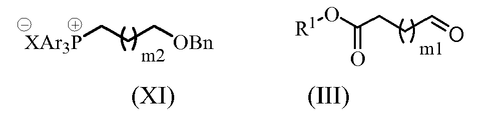 wherein
R¹ is selected from a substituted or unsubstituted C₁-C₂₀ alkyl,
m1 is an integer which is selected from 0, 1, 2, 3, 4 and 5;
m2 is an integer between 0 and 13;
X is a halogen; and
each of the Ar groups is independently selected from among the C₆-C₁₀ aryl groups.

Experimentally, the treatment of a compound of formula (XI) with a base leads to the formation of the corresponding ylide, which reacts with the compound of formula (III) to obtain a compound of formula (IV).

Preferably, the base used is *n-*BuLi, the aryl is phenyl and the halogen is Br.

It is therefore possible to prepare a compound of formula (VI) by means of the following synthetic sequence:
(i) reacting a compound of formula (III) with a compound of formula (XI) to yield a compound of formula (IV)
(ii) reacting said compound of formula (IV) with an oxalate of formula (XII) in the presence of a base to yield a compound of formula (V); and
(iii) reacting said compound of formula (V) with a phosphonate of formula (XIII).

The process for the synthesis of a compound of formula (XI) was performed by following the methodology described in Garcia, J.; López, M.; Romeo, J. Tetrahedron: Asymmetry 1999, 10, 2617-2626, which is incorporated in its entirety by reference. The process comprises the following steps
(i) reacting a benzylating agent in the presence of a base with a compound of formula (XXI) 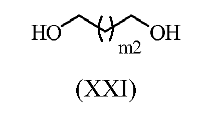 wherein
   m2 is an integer between 0 and 13; to obtain a compound of formula (XXII) 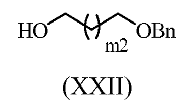
(ii) reacting said compound of formula (XXII) with a halogenating agent to obtain a compound of formula (XXV) 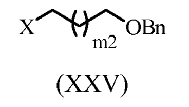 wherein
   m2 is an integer between 0 and 13; and
   X is a halogen atom;
   and
(iii) reacting said compound of formula (XXV) in the presence of a triarylphosphorus of formula PAr₃ to obtain said compound of formula (XI).

According to a particular embodiment, the compound of formula (XXI) is 1,12-dodecanediol. Preferably, the deprotonation of the latter with a base, such as NaH for example, followed by treatment with a benzylating agent, such as BnBr for example, leads to 12-benzyloxy-1-dodecanol **(6).** Then, in the compound **(6),** the alcohol is substituted with a halogen. The halogen is preferably bromine. This bromination reaction can be carried out in the presence of PPh₃ and CBr₄.

The halogenated compound of formula (X) is preferably transformed into a compound of formula (XI) by treatment with triphenylphosphine.

The compounds of formula (III) can be prepared by means of the oxidation of alcohols of formula (IIa) 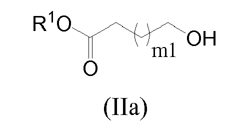 wherein
R¹ is selected from a substituted or unsubstituted C₁-C₂₀ alkyl; and
m1 is an integer which is selected from 0, 1, 2, 3, 4 and 5;

Oxidizing agents for the transformation of alcohols into aldehydes are known by the person skilled in the art (Larock, R. C. Comprehensive Organic transformations; John Wiley & Sons: New York, 1999, pp.: 1234-1249). According to a preferred embodiment, said oxidizing agent is selected from the group consisting of PCC (pyridinium chlorochromate), MnO₂, and DMSO/(COCl)₂/Et₃N, preferably PCC.

Said compounds of formula (IIa) can be prepared from compounds of formula (XXIVa) 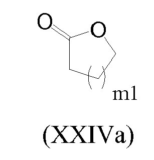 wherein
m1 is an integer which is selected from 0, 1, 2, 3, 4 and 5;
according to the process described in Bosone, E.; Farina, P.; Guazzi, G.; Innocenti, S.; Marotta, V. Synthesis 1983, 942-944, in the presence of an alcohol of formula R¹OH, wherein R¹ has the meaning indicated above.

According to a particular embodiment, the treatment of ε-caprolactone (compound of formula (XXIVa) wherein m1 is 3) in acidic medium using as a solvent an alcohol of formula R¹OH, preferably wherein R¹ is a C₁-C₃ alkyl, more preferably methanol, leads to the formation of the corresponding compound of formula (IIa), wherein the substituent R¹ corresponds to the rest of the alcohol used as a solvent. This alcohol of formula (IIa) gives rise to the corresponding compound of formula (III) by means of an oxidation reaction. The oxidizing reagent is preferably PCC.

Alternatively, the side chain of a compound of formula (VIII) can be elongated to provide a compound of formula (VIII) with a longer chain. According to a preferred embodiment, in the process of the invention the compound of formula (VIII), its stereoisomers or mixtures thereof, is a compound of formula (VIIIa), its stereoisomers or mixtures thereof, 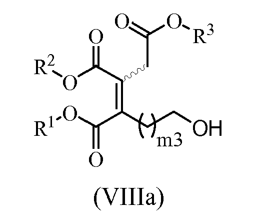 wherein
R¹, R² and R³ are independently selected from substituted or unsubstituted C₁-C₂₀ alkyl; and
m3 is an integer between 2 and 18;
it additionally comprises the following steps
(i) activating as a leaving group the hydroxyl group of a compound of formula (VIIIa), its stereoisomers or mixtures thereof, to form a compound of formula (XVIII), its stereoisomers or mixtures thereof 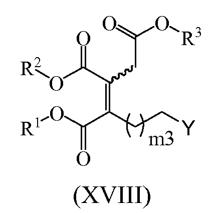 wherein
   m3, R¹, R² and R³ are as defined above; and
   Y is selected from -OSO₃H, OMs, -OTs, I and Br;
(ii) reacting said compound of formula (XVIII), its stereoisomers or mixtures thereof, with a compound of formula (X) 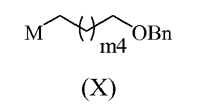 wherein
   M is a metal; and
   m4 is an integer between 0 and 18; and
   the relation m3 + m4 + 2= n is met; to obtain a compound of formula (VII), its stereoisomers or mixtures thereof 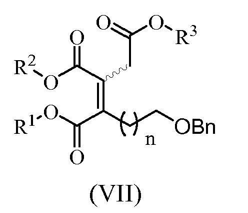 wherein
   n, R¹, R² and R³ are as defined above,
   and
(iii) subjecting said compound of formula (VII), its stereoisomers or mixtures thereof, to a hydrogenation reaction.

In the context of the present invention, preferred leaving groups are those which are active against organometallic reagents (nucleophilic groups) used in the formation of carbon-carbon bonds, preferably Grignard reagents transmetalated or non-transmetalated with Zn, Al, or Cu. Preferred leaving groups are -OTs, -I, -Br, Cl, - SO₃H. In a preferred embodiment, the leaving group is selected from Br and OTs.

In a preferred embodiment, the hydroxyl group is activated as the corresponding tosylate (OTs) in the presence of a base, such as pyridine for example.

According to another preferred embodiment, the treatment of a compound of formula (VIII) with CBr₄ and PPh₃ gives rise to the compound of formula (XVIII) wherein Y is Br.

The compounds of formula (XVI) can be prepared by reacting a compound of formula (XV) with a phosphonate of formula (XIII) in the presence of a base 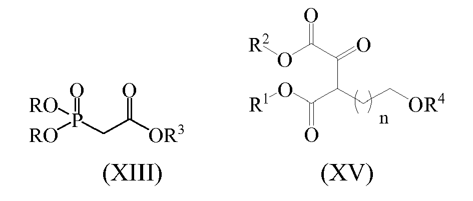 wherein
R is selected from C₁-C₄ alkyl substituted or unsubstituted with one or more halogen atoms, benzyl, phenyl and tolyl; and
R³ is selected from a substituted or unsubstituted C₁-C₂₀ alkyl; and
n, R¹, R² and R⁴ are as defined above.

The reactions of the compounds of formula (V) or (XV) with the phosphonates of formula (XIII) can give rise to mixtures of Z(cis)/E(trans)-isomers which can be separated, subjected to the following step as a mixture or enriched in the Z(cis)-isomer according to the process described below.

The compounds of formula (XV) can in turn be obtained by reacting an oxalate of formula (XII) with a compound of formula (XIV) 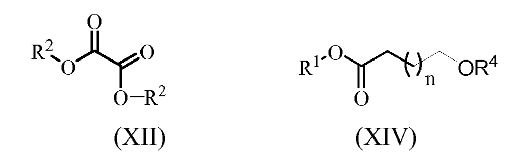 wherein
R² is selected from a substituted or unsubstituted C₁-C₂₀ alkyl; and
n, R¹ and R⁴ are as defined above.

As can be seen, the process followed for the synthesis of the compounds of formula (XIV) and (XV) is similar to the one used to synthesize the compounds of formula (V) and (VI), and the conditions can be the same or similar.

According to a particular embodiment, a compound of formula (XIV) is prepared by means of the treatment of an alcohol of formula (II) with a base and a silylating agent 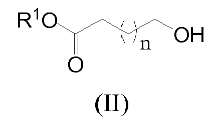 wherein

R¹ and n are as defined above.

Non-limiting examples of conditions under which this transformation for protecting the hydroxyl group can be carried out can be found in, for example, Dalla, V.; Catteau, J. P. Tetrahedron 1999, 55, 6497-6510, and the trialkylsilyl groups which can be used in this reaction, as well as reagents suitable for their introduction and removal, are known for the person skilled in the art (for example see Greene, T. W.; Wuts, P. G. M. Greene's Protective Groups in Organic Synthesis; John Wiley & Sons: Hoboken, 2007.

According to a particular embodiment, the base used is imidazole and the silylating agent is TBDMSC1.

According to another particular embodiment, the compounds of formula (II) can be prepared by reacting a compound of formula (XXIV) in the presence of an alcohol of formula R¹OH, wherein R¹ and n are as defined above

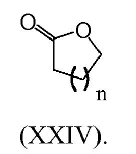

As can be seen, the process followed for the synthesis of the compounds of formula (II) is similar to the one used to synthesize the compounds of formula (IIa), and the conditions can be the same or similar.

An additional aspect of the present invention relates to a process for the synthesis of a compound of formula (I), 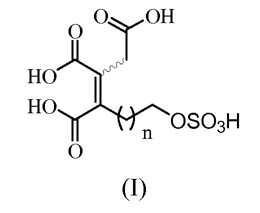 wherein n is an integer between 2 and 20;
its stereoisomers or mixtures thereof, characterized in that it comprises at least one of the following steps (ai), (aii) or (aiii)
(ai) subjecting a compound of formula (VI), its stereoisomers or mixtures thereof, to a hydro genation 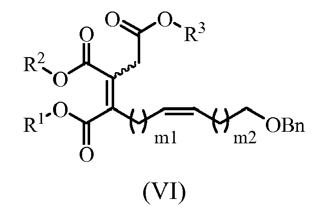 or
(aii) subjecting a compound of formula (VII), its stereoisomers or mixtures thereof, to a hydro genation 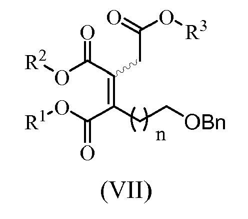 or
(aiii) removing the trialkylsilyl group of a compound of formula (XVI), its stereoisomers or mixtures thereof 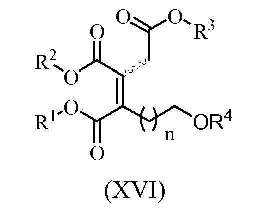 wherein
   n is an integer between 2 and 20;
   R¹, R² and R³ are independently selected from a substituted or unsubstituted C₁-C₂₀ alkyl;
   m1 is an integer which is selected from 0, 1, 2, 3, 4 and 5;
   m2 is an integer between 0 and 13; and
   R⁴ is a trialkylsilyl group;
to obtain a compound of formula (VIII), its stereoisomers or mixtures thereof 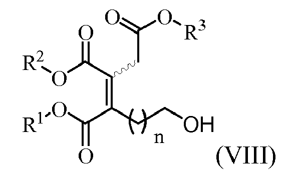 wherein
n, R¹, R² and R³ are as defined above;
and additionally comprises
(b) reacting said compound of formula (VIII), its stereoisomers or mixtures thereof, with a sulfating agent to yield a compound of formula (IX), its stereoisomers or mixtures thereof 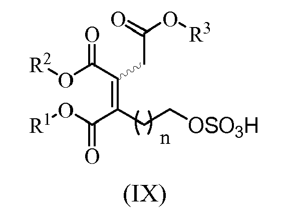 wherein
   n, R¹, R² and R³ are as defined above;
   and
(c) hydrolyzing the ester groups of said compound of formula (IX), its stereoisomers or mixtures thereof, to provide the corresponding acid groups.

Various sulfating agents are known for the person skilled in the art. The most common are SO₃ and HSO₃Cl. The sulfating agent is preferably the SO₃·pyridine complex.

The hydrolysis of ester groups to provide the corresponding acid groups is known by the person skilled in the art (see for example, Kocienski, P. J. Protecting Groups; Thieme: Stuttgart, 2000. pp.: 393-425). In this case, they would be conditions inert to the C3-C4 double bond and to the sulfate group present in the compounds of formula (IX). Non-limiting conditions for the purposes of the present invention are preferably those in which the hydrolysis of the compounds of formula (IX) is performed in the presence of an alkali metal or alkaline earth hydroxide, for example in the presence of LiOH, NaOH, Ba(OH)₂, or in the presence of Nₐ2S.

As has been indicated above, both C3-C4 stereoisomers of the compounds described in the present invention, as well as the mixture thereof are included within the present invention.

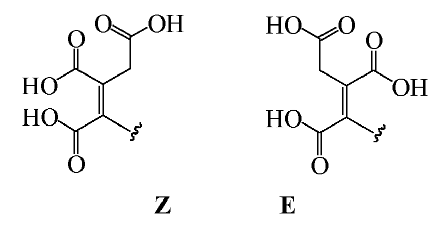

For example, the reduction of a compound of formula (VI)*-Z* leads to products with a Z configuration and the reduction of a compound of formula (VI)*-E* leads to products with a Z configuration.

However, it is possible to transform the E(trans)-isomer into the Z (cis) isomer into the corresponding Z(cis)-isomer or enrich a mixture of Z(cis)/E(trans)-isomers in the Z(cis)-isomer by means of exposure to a basic medium. Therefore, according to a preferred embodiment the process of the invention comprises obtaining a compound of formula cis-(I), cis-(VI), cis-(VII), cis-(VIII), cis-(VIIIa), cis-(IX), cis-(XVI), cis-(XVIa) or cis-(XVIII) or the enrichment in the cis-isomer of a mixture of cis- and trans-isomers of a compound of formula (I), (VI), (VII), (VIII), (VIIIa), (IX), (XVI), (XVIa) or (XVIII), by means of the exposure to a basic medium of a compound of formula trans-(I), trans-(VI), trans-(VII), trans-(VIII), trans-(VIIIa), trans-(IX), trans-(XVI), trans-(XVIa) or trans-(XVIII), or a mixture of cis- and trans-isomers of a compound of formula (I), (VI), (VII), (VIII), (VIIIa), (IX), (XVI), (XVIa) or (XVIII) . According to a preferred embodiment, the process of the invention comprises transforming a compound of formula trans-(VI), trans-(VII), trans-(IX) and/or trans-(XVI) into the corresponding compound of formula trans-(VI), trans-(VII), trans-(IX) and/or trans-(XVI), or in mixtures of the corresponding cis/trans-isomers. Said basic medium preferably comprises SO₃·pyridine.

### Compounds

As has already been indicated above, additional aspects of the present invention are compounds of formula (IV), (V), (VI), (VII), (XV), (XVI) and (XVIII), their stereoisomers or mixtures thereof, as defined above.

Another additional aspect is a compound of formula (VIII), its stereoisomers or mixtures thereof, as defined above with the exception of a compound of formula

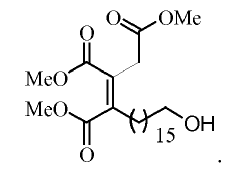

Another additional aspect is a compound of formula (I), its stereoisomers or mixtures thereof, as defined above, except

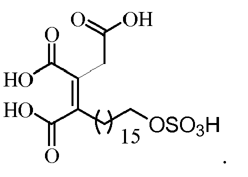

Another additional aspect is a compound of formula (IX), its stereoisomers or mixtures thereof, as defined above, except

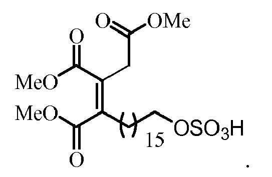

### Compounds of formula (VIIIb)

An additional aspect of the present invention is aimed at a process for preparing a compound of formula (VIIIb), which comprises reacting a compound of formula (XXX) with a hydride, preferably NaBH₄ (see Burke, S.D.; Danheiser, R.L. Handbook of Reagents for Organic Synthesis: Oxidizing and Reducing Agents; John Wiley & Sons: Chichester, 1999. pp.: 394-400). 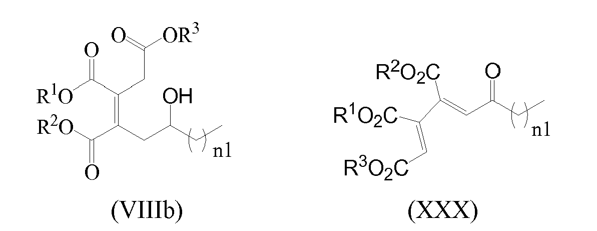 wherein
R¹, R² and R³ are independently selected from substituted or unsubstituted C₁-C₂₀ alkyl; and
n1 is an integer between 1 and 16, preferably between 3 and 12, more preferably between 5 and 10, more preferably 8;

According to a preferred embodiment, said compound of formula (XXX) is prepared by reacting a compound of formula (XXXI) with a compound of formula (XXXII) in the presence of a compound of formula PAr₃, preferably triphenylphosphine, wherein each of the Ar groups is independently selected from a C₆-C₁₀ aryl group, and wherein n, R¹, R² and R³ are as defined above. To see a non-limiting example of conditions for preparing compounds of formula (XXX), see Yavari, I.; Samzadeh-Kermani, A. R. Tetrahedron Lett. 1998, 39, 6343-6344.

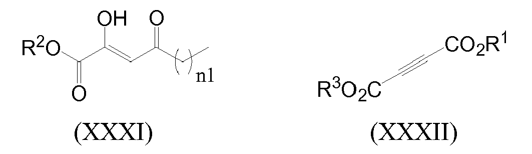

According to another preferred embodiment, said compound of formula (XXXI) is prepared by reacting a compound of formula (XXXIII) with an oxalate of formula (XII) in the presence of a base, wherein n1 and R² are as defined above.

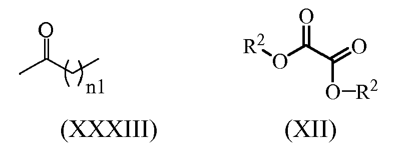

The compounds of formula (XXXI) can be obtained under the conditions described in Seki, K.; Isegawa, J.; Fukuda, M.; Ohki, M. Chem. Pharm. Bull. 1984, 32, 1568-1577; or Ashton, W. T.; Doss, G. A. J. Heterocycl. Chem. 1993, 30, 307-311, which are incorporated in their entirety by reference.

In short, this process allows obtaining the derivatives of the oreganic acid of formula (VIIIb) from commercial starting materials (compounds of formula (XXXIII) and (XII)) in a few steps and with a high yield.

### Examples

### Materials and methods

All the reactions were performed under an argon atmosphere, except those indicated in each case. The reagents and solvents used are from the companies Aldrich, Fluka, Merck, Sigma, Acros, Lancaster, SDS or Scharlau, and were purified by usual processes [Armarego, W. L.; Perrin, D. D. Purification of Laboratory Chemicals; Butterworth-Heinemann: Oxford, 1996] when necessary.

The solvents used were distilled and dried under an argon atmosphere. The dioxane was degassed by passing a stream of argon before being used.

The purification of the reaction products was performed by column chromatography under pressure (flash chromatography), using 60 Merck silica gel (with a 230-400 mesh particle size) as a stationary phase and previously distilled solvents as a mobile phase. The eluent used is indicated in each case and the ratios of the mixture of solvents used are always volume/volume.

The reactions were monitored by thin layer chromatography (TLC), using *60 F₂₅₄* silica gel chromatography plates marketed by Merck. The plates were developed using iodine vapors, 2% solution of 2,4-dinitrophenylhydrazine in EtOH (with 0.04% by volume of 97% H₂SO₄), 10% solution of phosphomolybdic acid in EtOH and UV light viewer (254 and 366 nm).

The general process used to create the H₂ atmosphere necessary for carrying out the hydrogenation reactions consisted of: after performing a vacuum in the reaction flask, a stream of H₂ was connected. The vacuum/H₂ process was repeated twice, and finally two balloons filled with H₂ were connected.

The reactions under high pressure conditions were carried out in Kimble brand glass vials.

The (fully decoupled) ¹H and ¹³C nuclear magnetic resonance spectra were performed at room temperature in the solvent indicated in each case (CDCl₃ and CD₃OD) using the following apparatuses: Varian Gemini-200 (200 MHz), Varian INOVA-300 (300 MHz), Bruker Avance-300 (300 MHz) and Varian INOVA-400 (400 MHz). The values of the chemical shifts are expressed in parts per million (δ, ppm), using as an internal reference the residual signal of the solvent: CDCl₃, 7.26 ppm (¹H-NMR) and 77.0 ppm (¹³C-NMR); CD₃OD, 3.31 ppm (¹H-NMR) and 49.0 ppm (¹³C-NMR).

The melting points (m.p.) were measured in a Reichert brand Kofler microscope.

The infrared (IR) spectra were recorded in the Perkin-Elmer spectrophotometer models 681 and FT-IR Spectrum One, and the frequencies (v) of the absorption maxima are expressed in cm⁻¹. The samples were analyzed as films between NaCl crystals or in KBr discs.

The low resolution mass spectra (LRMS) were recorded: (1) by direct injection of the sample into a Hewlett Packard 5973 MSD spectrophotometer using the electron impact (EI) ionization technique with an ionization energy of 70 eV; or (2) in a Hewlett Packard LCMS 1100 MSD spectrophotometer (an HPLC-coupled quadrupole analyzer) using the electrospray chemical ionization technique (API-ES) in positive or negative modes, applying a capillary voltage of 4000 V, a drying temperature of 330°C and using a [1:1] H₂O/MeOH mixture with 1% AcOH as a carrier. The data obtained are expressed in mass units (*m*/*z*) and the values in parenthesis correspond to the relative intensities with respect to the base peak (100%). The molecular peak is specified as M⁺.

The elemental analyses (E.A.) were performed with the Perkin-Elmer 240C and Heraus CHN-O-Rapid analyzers. The data calculated and observed are expressed in percentages.

### Example 1. Preparation of methyl 6-hydroxyhexanoate (4)

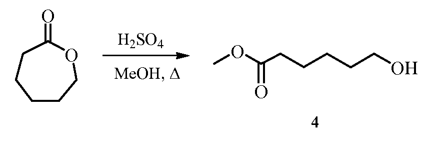

A solution of ε-caprolactone (25 g, 220 mmoles) in MeOH (25 ml) was added to a solution of 97% H₂SO₄ (2.5 ml) in MeOH (25 ml). The mixture was heated under reflux for 30 minutes. After that time, the mixture was neutralized with an aqueous solution of 10% NaOH (17.5 ml) and extracted with AcOEt (3 × 25 ml). The organic phase was dried with anhydrous Na₂SO₄, filtered and the solvent was removed under reduced pressure, obtaining (32 g, quantitative yield) methyl 6-hydroxyhexanoate (**4**), which was used in the following step without purifying.
**¹H-NMR** (200 MHz, CDCl₃). δ 3.55 (3H, s, -OCH₃), 3.53 (2H, t, *J* = 6.2 Hz, H-6), 2.22 (2H, t, *J*= 7.2 Hz, H-2),1.62-1.23 (6H, m, -CH₂-).

### Example 2. Preparation of methyl 5-formylpentanoate (5)

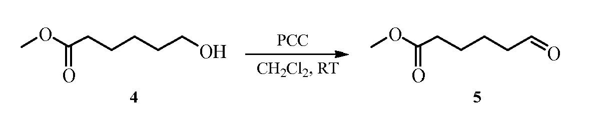

PCC (2.65 g, 12.3 mmoles) was added to a solution of methyl 6-hydroxyhexanoate **(4)** (1.2 g, 8.19 mmoles) in CH₂Cl₂ (50 ml). The mixture was stirred at room temperature for 2.5 hours. After that time, the solvent was removed under reduced pressure. The residue was suspended in a 1:1 hexane/AcOEt mixture (30 ml) and filtered through silica gel to remove the remains of the reagent, obtaining (1.16 g, quantitative yield) methyl 5-formylpentanoate **(5),** which was used in the following step without purifying.
**¹H-NMR** (200MHz, CDCl₃). δ 9.71 (1H, m, H-6), 3.61 (3H, s, -OCH₃), 2.39 (4H, m, H-2, H-5), 1.65 (4H, m, H-3, H-4).

### Example 3. Preparation of 12-benzyloxy-1-dodecanol (6)

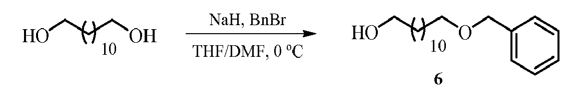

A solution of 1,12-dodecanediol (5.0 g, 24.7 mmoles) in a 2:1 THF/DMF mixture (36 ml) was added to a suspension of NaH (0.595 g, 24.83 mmoles) in DMF (18 ml) at 0°C. The mixture was stirred at 0°C for 2 hours. After that time, BnBr (4.42 g, 25.8 mmoles) was added at 0°C. The resulting mixture was stirred at room temperature for 4 hours. Then, the mixture was cooled at 0°C and H₂O (75 ml) was added. The phases were separated, and the aqueous phase was extracted with Et₂O (5 × 20 ml). The organic phase was washed with sat. NaCl (30 ml), dried with anhydrous MgSO₄, filtered and the solvent was removed under reduced pressure. The product was purified by a chromatographic column (hexane/AcOEt, 10:1), obtaining (3.59 g, yield 50%) 12-benzyloxy-1-dodecanol **(6),** as a transparent oil.
**¹H-NMR** (200MHz, CDCl₃). δ 7.36-7.27 (5H, m, Ar-H), 4.51 (2H, s, -OCH₂Ph), 3.60 (2H, t, *J*= 6.5 Hz), 3.45 (2H, t, *J*= 6.4 Hz), 1.60 (4H, m), 1.40 (16H, m).

### Example 4. Preparation of 12-benzyloxy-1-bromododecane (7)

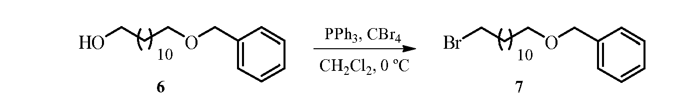

CBr₄ (3.34 g, 10.08 mmoles) was added to a solution of 12-benzyloxy-1-dodecanol (**6**) (2.36 g, 8.06 mmoles) and PPh₃ (2.64 g, 10.08 mmoles) in CH₂Cl₂ (40 ml) at 0°C. The mixture was stirred at room temperature for 30 minutes. After that time, the solvent was removed under reduced pressure. The residue was suspended in hexane (115 ml) and stirred at room temperature for 30 minutes. After that time, the mixture was filtered under vacuum and the solvent was removed under reduced pressure. The product was purified by a chromatographic column (hexane/CH₂Cl₂, 2:1), obtaining (2.45 g, yield 86%) 12-benzyloxy-1-bromododecane **(7),** as a transparent oil.
**¹H-NMR** (200MHz, CDCl₃). δ 7.35 (5H, m, Ar-H), 4.48 (2H, s, -OCH₂Ph), 3.45 (2H, t, *J* = 6.5 Hz), 3.35 (2H, t, *J* = 6.5 Hz), 1.85 (2H, m, -CH₂-), 1.58 (2H, m, -CH₂-), 1.36-1.15 (16H, m, -CH₂-).

### Example 5. Preparation of [12-benzyloxydodec-1-yl]triphenylphosphonium bromide (8)

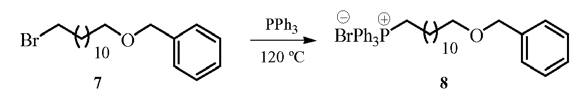

A mixture of 12-benzyloxy-1-bromododecane **(7)** (1.58 g, 4.44 mmoles) and PPh₃ (1.28 g, 4.89 mmoles) was heated in a Kimble at 120°C for 3 hours. After that time, the product was purified by a chromatographic column (CH₂Cl₂/MeOH, 9:1), obtaining (2.5 g, yield 91%) [12-benzyloxydodec-1-yl]triphenylphosphonium bromide (**8**), as a transparent oil.
**¹H-NMR** (200MHz, CDCl₃). δ 7.70 (15H, m), 7.22 (5H, m, Ar-H), 4.52 (2H, s, - OCH₂Ph), 3.80 (2H, m, H-1), 3.38 (2H, t, *J*= 6.5 Hz, H-12), 1.55 (4H, m, -CH₂-), 1.36-1.15 (16H, m, -CH₂-).

### Example 6. Preparation of methyl 6-(tert-butyldimethylsilyloxy)hexanoate (24)

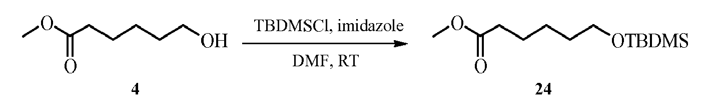

A solution of TBDMSC1 (7.16 g, 46.08 mmoles) in DMF (10 ml) was added to a solution of methyl 6-hydroxyhexanoate **(4)** (5.613 g, 38.40 mmoles) and imidazole (3.13 g, 46.08 mmoles) in DMF (50 ml) at 0°C. The mixture was stirred at room temperature for 24 hours. After that time, H₂O (80 ml) and AcOEt (40 ml) were added. The phases were separated, and the aqueous phase was extracted with AcOEt (3 × 20 ml). The organic phase was washed with sat. CuSO₄ (2 × 30 ml) and sat. NaCl (2 × 30 ml), dried with anhydrous Na₂SO₄, filtered and the solvent was removed under reduced pressure. The product was purified by a chromatographic column (hexane/AcOEt, 25:1), obtaining (6.46 g, yield 73%) methyl 6-(*tert*-butyldimethylsilyloxy)hexanoate **(24)** as a colorless oil.
**¹H-NMR** (200MHz, CDCl₃). δ 3.56 (3H, s, -OCH₃), 3.49 (3H, t, *J* = 6.2 Hz, H-6), 2.21 (2H, t, *J*= 7.6 Hz, H-2), 1.61-1.20 (6H, m, -CH₂-), 0.78 (9H, s, *tert*-BuSi), -0.06 (6H, s, (Me)₂Si).

### Example 7. Preparation of methyl rac-(R)-7-(tert-butyldimethylsilyloxy)-3-(methoxycarbonyl)-2-oxoheptanoate (25)

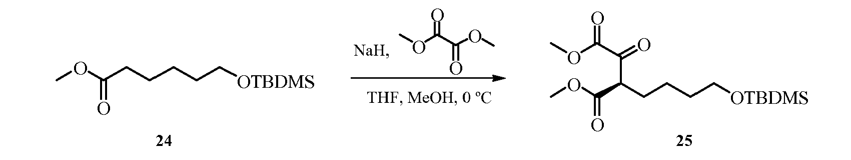

MeOH (0.23 ml) was added to a suspension ofNaH (0.507 g, 21.11 mmoles) in THF (9.2 ml) at 0°C. The mixture was stirred until it reached room temperature. Then, dimethyl oxalate (2.29 g, 19.19 mmoles) and methyl 6-(*tert-*butyldimethylsilyloxy)hexanoate **(24)** (5.0 g, 19.19 mmoles) were added. The resulting mixture was heated under reflux for 3 hours. After that time, H₂O (10 ml) and an aqueous solution of 10% HCl were added until neutral pH. The phases were separated, and the aqueous phase was extracted with AcOEt (3 × 10 ml). The organic phase was dried with anhydrous Na₂SO₄, filtered and the solvent was removed under reduced pressure. The product was purified by a chromatographic column (hexane/AcOEt, 25:1), obtaining (4.41 g, yield 67%) methyl *rac*-(*R*)-7-(*tert*-butyldimethylsilyloxy)-3-(methoxycarbonyl)-2-oxoheptanoate **(25),** as a transparent oil.
**¹H-NMR** (200MHz, CDCl₃). δ 3.92 (1H, t, *J*= 6.8 Hz, H-3), 3.78 (3H, s, -OCH₃), 3.62 (3H, s, -OCH₃), 3.49 (2H, t, *J* = 5.9 Hz, H-7), 1.87-1.41 (6H, m, -CH₂-), 0.77 (9H, s, *tert*-BuSi), -0.07 (6H, s, (Me)₂Si).

### Example 8. Reaction of methyl rac-(R)-7-(tert-butyldimethylsilyloxy)-3-(methoxycarbonyl)-2-oxoheptanoate (25) with methyl (dimethoxyphosphoryl)acetate

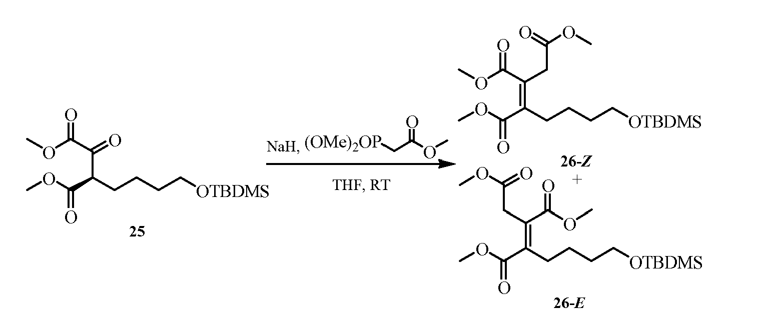

A solution of methyl (dimethoxyphosphoryl)acetate (0.105 g, 0.57 mmoles) in THF (0.3 ml) was added to a suspension of NaH (0.013 g, 0.57 mmoles) in THF (2.3 ml). The mixture was stirred at room temperature until H₂ was no longer evolved. Then, a solution of methyl *rac*-(*R*)-7-(*tert*-butyldimethylsilyloxy)-3-(methoxycarbonyl)-2-oxoheptanoate **(25)** (0.100 g, 0.29 mmoles) in THF (0.3 ml) was added. The resulting mixture was stirred at room temperature for 24 hours. After that time, Celite was added and the solvent was removed under reduced pressure. The product was purified by a chromatographic column (hexane/AcOEt, 15:1), obtaining (0.063 g, yield 54%) methyl (*Z*)-8-(*tert*-butyldimethylsilyloxy)-3,4-bis(methoxycarbonyl)-3-octenoate **(26*-Z*)** and (0.024 g, yield 21%) methyl (*E*)-8-(*tert*-butyldimethylsilyloxy)-3,4-bis(methoxycarbonyl)-3-octenoate (26-*E*), both as a transparent oil.

### Methyl (Z)-8-(tert-butyldimethylsilyloxy)-3,4-bis(methoxycarbonyl)-3-octenoate (26-Z)

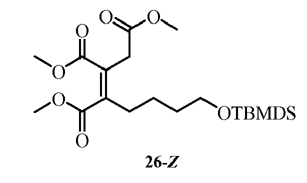

**¹H-NMR** (200MHz, CDCl₃). δ 3.67 (3H, s, -OCH₃), 3.62 (3H, s, -OCH₃), 3.57 (3H, s, - OCH₃), 3.48 (2H, m, H-8), 3.29 (2H, s, H-2), 2.26 (2H, m, H-5), 1.40 (4H, m, -CH₂-), 0.76 (9H, s, *tert*-BuSi), -0.08 (6H, s, (Me)₂Si).

### Methyl (E)-8-(tert-butyldimethylsilyloxy)-3,4-bis(methoxycarbonyl)-3-octenoate (26-E)

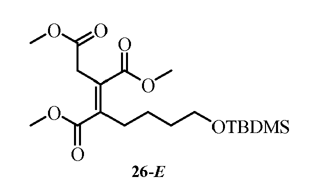

**¹H-NMR** (200MHz, CDCl₃). δ 3.67 (6H, s, -OCH₃), 3.57 (3H, s, -OCH₃), 3.49 (2H, m, H-8), 3.41 (2H, s, H-2), 2.53 (2H, m, H-5), 1.41 (4H, m, -CH₂-), 0.77 (9H, s, *tert*-BuSi), -0.07 (6H, s, (Me)₂Si).

### Example 9. Preparation of methyl (Z)-8-hydroxy-3,4-bis(methoxycarbonyl)-3-octenoate (27-Z)

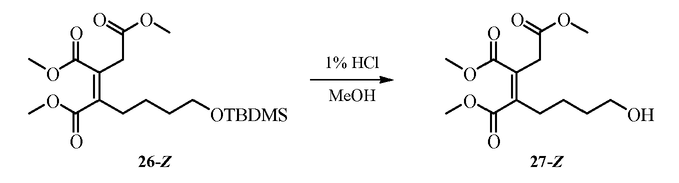

A solution of methyl (*Z*)-8-(*tert*-butyldimethylsilyloxy)-3,4-bis(methoxycarbonyl)-3-octenoate **(26*-Z*)** (0.100 g, 0.24 mmoles) in HCl (1% in MeOH, 1 ml) was stirred at room temperature for 2 hours. After that time, the mixture was neutralized with an aqueous solution of 10% NaHCO₃ (1 ml), and CH₂Cl₂ (2 ml) was added. The phases were separated, and the aqueous phase was extracted with CH₂Cl₂ (3 × 2 ml). The organic phase was washed with sat. NaCl (2 ml), dried with anhydrous Na₂SO₄, filtered and the solvent was removed under reduced pressure, obtaining (0.065 g, yield 95%) methyl (*Z*)-8-hydroxy-3,4-bis(methoxycarbonyl)-3-octenoate **(27*-Z*)** as a colorless oil.
**¹H-NMR** (200 MHz, CDCl₃). δ 3.79 (3H, s, -OCH₃), 3.73 (3H, s, -OCH₃), 3.69 (3H, s, - OCH₃), 3.63 (2H, t, *J* = 6.0 Hz, H-8), 3.42 (2H, s, H-2), 2.40 (2H, t, *J* = 7.4 Hz, H-5), 1.58 (4H, m, -CH₂-).

### Example 10. Preparation of methyl (Z)-3,4-bis(methoxycarbonyl)-8-sulfooxy-3-octenoate (31-Z)

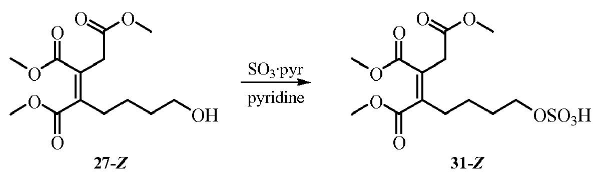

SO₃·pyr (0.276 g, 1.73 mmoles) was added in small portions to a solution of methyl (Z)-8-hydroxy-3,4-bis(methoxycarbonyl)-3-octenoate **(27*-Z*)** (0.100 g, 0.34 mmoles) in pyridine (6.5 ml). The mixture was stirred at room temperature for 24 hours. After that time, the solvent was removed under reduced pressure. The residue was suspended in a 1:1 H₂O/MeOH mixture (2 ml), neutralized with an aqueous solution of 0.5M KOH and the solvent was removed under reduced pressure. The product was purified by trituration with MeOH, obtaining (0.126 g, quantitative yield) methyl (Z)-3,4-bis(methoxycarbonyl)-8-sulfooxy-3-octenoate **(31*-Z*),** as a white solid.
**IR** (BrK): ν 3457, 3075, 2955, 1726, 1638, 1489, 1437, 1261, 1201, 1008, 756, 683 cm⁻¹.
**¹H-NMR** (200 MHz, CDCl₃). δ 4.11 (2H, t, *J*= 6.0 Hz, H-8), 3.78 (3H, s, -OCH₃), 3.73 (3H, s, -OCH₃), 3.69 (3H, s, -OCH₃), 3.42 (2H, s, H-2), 2.40 (2H, t, *J* = 7.4 Hz, H-5), 1.60 (4H, m, -CH₂-).

### Example 11. Preparation of methyl (Z)-3,4-bis(methoxycarbonyl)-8-(p-toluenesulfonyl)-3-octenoate (32-Z)

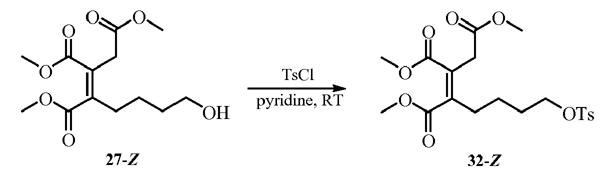

A solution of methyl (Z)-8-hydroxy-3,4-bis(methoxycarbonyl)-3-octenoate **(27-Z)** (0.100 g, 0.347 mmoles) in pyridine (1 ml) was added to a solution of TsCl (0.099 g, 0.52 mmoles) in pyridine (1 ml) at 0°C. The mixture was stirred at 0°C for 2 hours. After that time, H₂O (4 ml) was added. The phases were separated, and the aqueous phase was extracted with Et₂O (3 × 4 ml). The organic phase was washed with an aqueous solution of 10% HCl (× 2, until an acidic pH), with H₂O (4 ml), with sat. NaHCO₃ (4 ml) and with sat. NaCl (4 ml), dried with anhydrous MgSO₄, filtered and the solvent was removed under reduced pressure, obtaining (0.110 g, yield 72%) methyl (*Z*)-3,4-bis(methoxycarbonyl)-8-(*p*-toluenesulfonyl)-3-octenoate **(32*-Z*),** as a colorless oil.
**¹H-NMR** (200 MHz, CDCl₃). δ 7.79 (2H, d, *J*= 8.3 Hz, Ar-H), 7.36 (2H, d, *J*= 8.3 Hz, Ar-H), 4.02 (2H, t, *J* = 6.1 Hz, H-8), 3.79 (3H, s, -OCH₃), 3.75 (3H, s, -OCH₃), 3.71 (3H, s, -OCH₃), 3.39 (2H, s, H-2), 2.47 (3H, s, -CH₃), 2.36 (2H, t, *J* = 7.6 Hz, H-5), 1.80-1.45 (4H, m, -CH₂-).

### Example 12. Preparation of methyl (Z)-8-bromo-3,4-bis(methoxycarbonyl)-3-octenoate (29-Z)

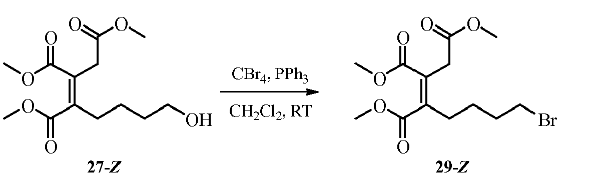

A solution of PPh₃ (0.341 g, 1.30 mmoles) in CH₂Cl₂ (2 ml) was added to a solution of methyl (Z)-8-hydroxy-3,4-bis(methoxycarbonyl)-3-octenoate **(27*-Z*)** (0.300 g, 1.04 mmoles) and CBr₄ (0.431 g, 1.30 mmoles) in CH₂Cl₂ (6.5 ml) at 0°C. The mixture was stirred at room temperature for 2 hours. After that time, Celite was added and the solvent was removed under reduced pressure. The product was purified by a chromatographic column (hexane/AcOEt, 4:1), obtaining (0.218 g, yield 60%) methyl (Z)-8-bromo-3,4-bis(methoxycarbonyl)-3-octenoate **(29*-Z*),** as a colorless oil.
**¹H-NMR** (200 MHz, CDCl₃). δ 3.80 (3H, s, -OCH₃), 3.74 (3H, s, -OCH₃), 3.70 (3H, s, - OCH₃), 3.42 (2H, s, H-2), 3.39 (2H, t, *J* = 6.6 Hz, H-8), 2.39 (2H, t, *J* = 7.9 Hz, H-5), 1.88 (2H, m, -CH₂-), 1.62 (2H, m, -CH₂-).

### Example 13. Preparation of methyl (Z)-18-benzyloxy-6-octadecenoate (13)

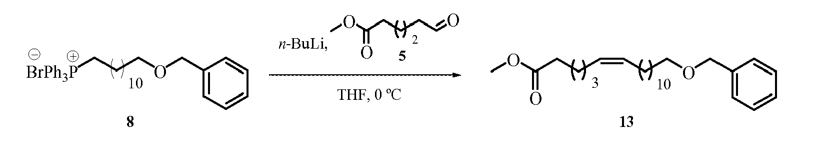

*n*-BuLi (0.545 g, 8.52 mmoles) was added to a solution of [12-benzyloxydodec-1-yl]triphenylphosphonium bromide **(8)** (3.71 g, 6.0 mmoles) in THF (85 ml) at 0°C. The mixture was stirred at 0°C for 45 minutes. After that time, a solution of methyl 5-formylpentanoate **(5)** (0.952 g, 8.34 mmoles) in THF (2 ml) was added at 0°C. The mixture was stirred at room temperature for 1 hour. After that time, the reaction mixture was cooled at 0°C and sat. NH₄Cl was added (30 ml). The phases were separated, and the aqueous phase was extracted with Et₂O (4 × 15 ml). The organic phase was dried with anhydrous MgSO₄, filtered and the solvent was removed under reduced pressure. The product was purified by a chromatographic column (hexane/AcOEt, 20:1), obtaining (2.61 g, yield 53%) methyl (*Z*)-18-benzyloxy-6-octadecenoate **(13),** as a transparent oil.
**IR** (NaCl): ν 3004, 2926, 2854, 1741, 1496, 1454, 1435, 1362, 1204, 1170, 1102, 1028, 734, 697 cm⁻¹.
**¹H-NMR** (300MHz, CDCl₃). δ 7.30 (5H, m, Ar-H), 5.39 (1H, dt, *J*= 7.0, 10.9 Hz, H-6 or H-7), 5.30 (1H, dt, *J*= 8.3, 10.9 Hz, H-7 or H-6), 4.50 (2H, s, CH₂Ph), 3.66 (3H, s, - OCH₃), 3.46 (2H, t, *J*= 6.5 Hz, H-18), 2.31 (2H, t, *J*= 7.5 Hz, H-2), 1.96 (4H, m, H-5, H-8), 1.63 (4H, m, -CH₂-), 1.34 (18H, m, -CH₂-).
**¹³C-NMR** (50MHz, CDCl₃). δ 174.1, 138.6, 130.9, 130.4, 129.0, 128.2, 127.5, 127.3, 72.7, 70.4, 51.3, 33.9, 29.7, 29.6, 29.5, 29.5, 29.4, 29.2, 29.1, 29.0, 27.1, 26.7, 26.1, 24.5, 24.3.
**LRMS(EI):** *m*/*z* 402(M⁺, 2), 371(1), 342(0), 311(14), 279(37), 261(16), 195(3), 91(100).
**E.A.** (C₂₆H₄₂O₃): Found: C, 77.50, H, 10.45; Calculated: C, 77.56, H, 10.51.

### Example 14. Preparation of methyl rac-(Z,R)-19-benzyloxy-3-(methoxycarbonyl)-2-oxo-7-nonadecenoate (22)

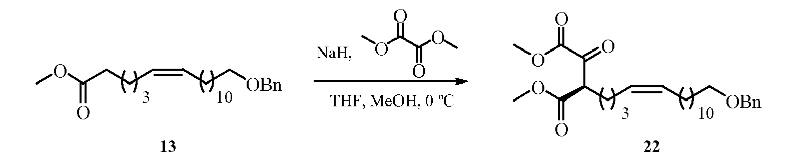

MeOH (0.022 ml) was added to a suspension ofNaH (0.044 g, 1.87 mmoles) in THF (1 ml) at 0°C. The mixture was stirred until it reached room temperature. Then, dimethyl oxalate (0.221 g, 1.87 mmoles) and methyl (Z)-18-benzyloxy-6-octadecenoate **(13)** (0.685 g, 1.70 mmoles) were added. The resulting mixture was heated under reflux for 4 hours. After that time, H₂O (10 ml) and an aqueous solution of 10% HCl were added until neutral pH. The phases were separated, and the aqueous phase was extracted with AcOEt (3 × 7 ml). The organic phase was dried with anhydrous Na₂SO₄, filtered and the solvent was removed under reduced pressure. The product was purified by a chromatographic column (hexane/AcOEt, 15:1), obtaining (0.70 g, yield 85%) methyl *rac*-(*Z,R*)-19-benzyloxy-3-(methoxycarbonyl)-2-oxo-7-nonadecenoate **(22),** as a transparent oil.
**IR** (NaCl): ν 3454, 3005, 2926, 2853, 1735, 1657, 1495, 1454, 1436, 1361, 1249, 1100, 815, 735, 697 cm⁻¹.
**¹H-NMR** (300MHz, CDCl₃). δ 7.29 (5H, m, Ar-H), 5.37 (1H, dt, *J*= 6.1, 10.7 Hz, H-7 or H-8), 5.31 (1H, dt, *J*= 6.8, 10.7 Hz, H-8 or H-7), 4.50 (2H, s, -CH₂Ph), 4.03 (1H, t, *J* = 6.8 Hz, H-3), 3.88 (3H, s, -OCH₃), 3.72 (3H, s, -OCH₃), 3.46 (2H, t, *J* = 6.5 Hz, H-19), 1.97 (6H, m, -CH₂-), 1.65-1.23 (20H, m, -CH₂-).
**¹³C-NMR** (75MHz, CDCl₃). δ 188.5, 169.3, 160.7, 138.6, 130.8, 130.3, 128.2, 127.4, 127.3, 72.7, 70.4, 53.7, 53.1, 52.4, 29.6, 29.5, 29.4, 29.4, 29.3, 29.2, 29.0, 27.1, 27.0, 26.9, 26.7, 26.6, 26.1.
**LRMS(EI):** *m*/*z* 488(M⁺, 0), 457(0), 397(2), 347(8), 287(30), 237(9), 91(100).
**E.A.** (C₂₉H₄₄O₆): Found: C, 71.20, H, 9.00; Calculated: C, 71.28, H, 9.08.

### Example 15. Reaction of methyl rac-(Z,R)-19-benzyloxy-3-(methoxycarbonyl)-2-oxo-7-nonadecenoate (22) with methyl (dimethoxyphosphoryl)acetate

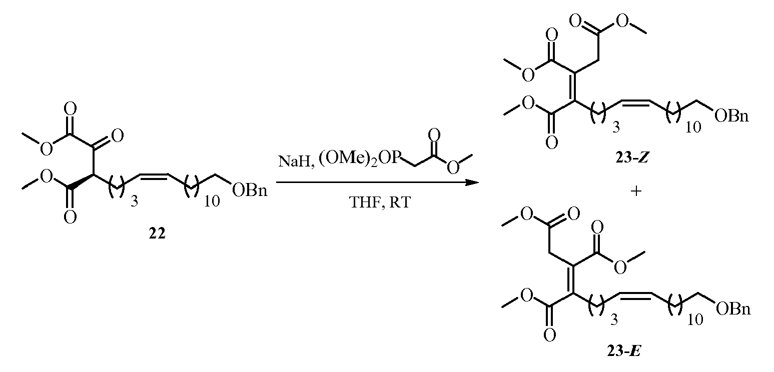

A solution of methyl (dimethoxyphosphoryl)acetate (1.11 g, 6.12 mmoles) in THF (3.2 ml) was added to a suspension of NaH (0.147 g, 6.12 mmoles) in THF (10.6 ml). The mixture was stirred at room temperature until H₂ was no longer evolved. Then, a solution of methyl *rac*-(*Z,R*)-19-benzyloxy-3-(methoxycarbonyl)-2-oxo-7-nonadecenoate **(22)** (1.36 g, 2.78 mmoles) in THF (3.2 ml) was added. The resulting mixture was stirred at room temperature for 24 hours. After that time, Celite was added and the solvent was removed under reduced pressure. The product was purified by a chromatographic column (hexane/AcOEt, 10:1), obtaining (0.754 g, yield 50%) methyl (3Z,8Z)-20-benzyloxy-3,4-bis(methoxycarbonyl)-3,8-eicosadienoate **(23*-Z*)** and (0.261 g, yield 18%) methyl (3*E*,8*Z*)-20-benzyloxy-3,4-bis(methoxycarbonyl)-3,8-eicosadienonate **(23-*E*)**, both as a transparent oil.

### Methyl (3Z,8Z)-20-benzyloxy-3,4-bis(methoxycarbonyl)-3,8-eicosadienoate (23-Z)

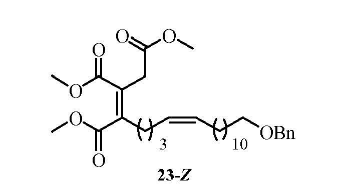

**IR (NaCl):** ν 3448, 3003, 2926, 2853, 1735, 1645, 1495, 1454, 1435, 1314, 1268, 1198, 1169, 1099, 1027, 797, 736, 698 cm⁻¹.
**¹H-NMR** (300MHz, CDCl₃). δ 7.29 (5H, m, Ar-H), 5.39 (1H, dt, *J*= 6.6, 10.5 Hz, H-8 or H-9), 5.35 (1H, dt, *J* = 6.8, 10.5 Hz, H-9 or H-8), 4.50 (2H, s, -OCH₂Ph), 3.79 (3H, s, -OCH₃), 3.73 (3H, s, -OCH₃), 3.69 (3H, s, -OCH₃), 3.46 (2H, t, *J*= 6.5 Hz, H-20), 3.40 (2H, s, H-2), 2.34 (2H, m, -CH₂-), 2.02 (4H, m, -CH₂-), 1.65-1.23 (20H, m, -CH₂-).
**¹³C-NMR** (75MHz, CDCl₃). δ 169.7, 169.3, 166.2, 145.6, 138.4, 130.9, 128.5, 127.9, 127.9, 127.2, 125.4, 72.5, 70.1, 52.0, 51.8, 51.8, 33.4, 30.5, 29.5, 29.4, 29.3, 29.2, 29.2, 29.0, 27.2, 27.0, 26.4, 25.9.
**LRMS(EI):** *m*/*z* 544(M⁺, 0), 512(2), 485(2), 453(2), 389(23), 329(8), 198(45), 166(50), 91(100).
**E.A.** (C₃₂H₄₈O₇): Found: C, 70.45, H, 9.00; Calculated: C, 70.56, H, 8.88.

### Methyl (3E,8Z)-20-benzyloxy-3,4-bis(methoxycarbonyl)-3,8-eicosadienoate (23-E)

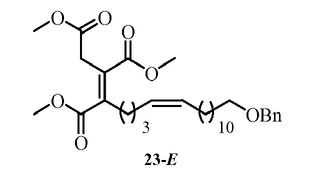

**IR** (NaCl)**:** ν 3436, 3000, 2926, 2853, 1741, 1435, 1251, 1201, 1169, 1101, 1014, 735, 697 cm⁻¹.
**¹H-NMR** (300MHz, CDCl₃). δ 7.31 (5H, m, Ar-H), 5.37 (1H, dt, *J*= 7.0, 10.9 Hz, H-8 or H-9), 5.33 (1H, dt, *J* = 8.3, 10.9 Hz, H-9 or H-8), 4.50 (2H, s, -OCH₂Ph), 3.78 (6H, s, -OCH₃), 3.68 (3H, s, -OCH₃), 3.51 (2H, s, H-2), 3.46 (2H, t, *J* = 6.5 Hz, H-20), 2.58 (2H, m, -CH₂-), 2.07 (4H, m, -CH₂-), 1.65-1.23 (20H, m, -CH₂-).
**¹³C-NMR** (75MHz, CDCl₃). δ 170.4, 168.6, 167.4, 146.5, 143.7, 138.6, 131.0, 130.6, 128.7, 128.5, 128.1, 127.4, 127.2, 72.7, 70.3, 51.9, 51.8, 51.6, 36.4, 29.6, 29.5, 29.4, 29.4, 29.3, 29.2, 29.0, 28.5, 27.1, 26.8, 26.0.
**LRMS(EI):** *m*/*z* 544(M⁺, 0), 512(1), 453(3), 421(8), 389(26), 357(13), 198(21), 91(100).
**E.A.** (C₃₂H₄₈O₇): Found: C, 70.50, H, 9.05; Calculated: C, 70.56, H, 8.88.

### Example 16. Preparation of methyl (Z)-20-hydroxy-3,4-bis(methoxycarbonyl)-3-eicosadienoate (33-Z)

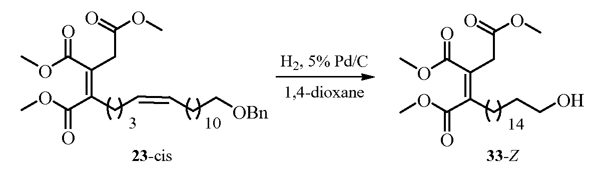

Pd/C (0.042 g, 5% by weight, 0.02 mmoles) was added to a solution of methyl (3Z,8Z)-20-benzyloxy-3,4-bis(methoxycarbonyl)-3,8-eicosadienoate **(23*-Z*)** (0.217 g, 0.398 mmoles) in 1,4-dioxane (4.33 ml). The mixture was stirred under an argon atmosphere at room temperature for 1 hour. After that time, the mixture was filtered through Celite with AcOEt and the solvent was removed under reduced pressure. The product was purified by a chromatographic column (hexane/AcOEt, 3:1), obtaining (0.149 g, yield 82%) methyl (Z)-20-hydroxy-3,4-bis(methoxycarbonyl)-3-eicosenoate **(33*-Z*),** as a white solid.

### Example 17. Preparation of methyl (Z)-3,4-bis(methoxycarbonyl)-20-sulfooxy-3-eicosenoate (37-Z)

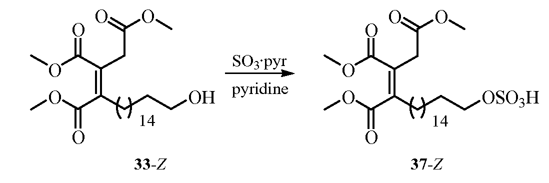

SO₃·pyr (0.181 g, 1.11 mmoles) was added in small portions to a solution of methyl (Z)-20-hydroxy-3,4-bis(methoxycarbonyl)-3-eicosenoate **(33*-Z*)** (0.102 g, 0.22 mmoles) in pyridine (4.2 ml). The mixture was stirred at room temperature for 2 hours. After that time, the solvent was removed under reduced pressure. The residue was suspended in a 1:1 H₂O/MeOH mixture (2 ml), neutralized with an aqueous solution of 0.5M KOH and the solvent was removed under reduced pressure. The product was purified by trituration with MeOH, obtaining (0.119 g, quantitative yield) methyl (Z)-3,4-bis(methoxycarbonyl)-20-sulfooxy-3-eicosenoate **(37*-Z*),** as a white solid. **m.p.:** 129-130°C.
**IR** (BrK): ν 3452, 2923, 2852, 1733, 1643, 1436, 1255, 1217, 1065, 1008, 773, 619, 577 cm⁻¹.
**¹H-NMR** (300 MHz, CDCl₃). δ 4.10 (2H, m, H-20), 3.79 (3H, s, -OCH₃), 3.73 (3H, s, - OCH₃), 3.69 (3H, s, -OCH₃), 3.41 (2H, s, H-2), 2.33 (4H, m, -CH₂-), 1.60-1.24 (26H, m, -CH₂-).
**¹³C-NMR** (75 MHz, CDCl₃). δ 170.2, 169.7, 166.7, 146.1, 125.4, 69.8, 52.3, 52.1, 33.7, 31.3, 29.9, 29.5, 29.3, 27.5, 25.8.
**LRMS(EI):** *m*/*z* 536(M⁺, 0), 518(13), 486(6), 458(10), 426(5), 346(7), 287(6), 198(44), 166(88), 55(100).
**E.A.** (C₂₅H₄₄O₁₀S): Found: C, 56.00, H, 8.30; Calculated: C, 55.95, H, 8.26.

### Example 18. Reaction of methyl (E)-20-hydroxy-3,4-bis(methoxycarbonyl)-3-eicosenoate (33-E) with SO₃·pyr

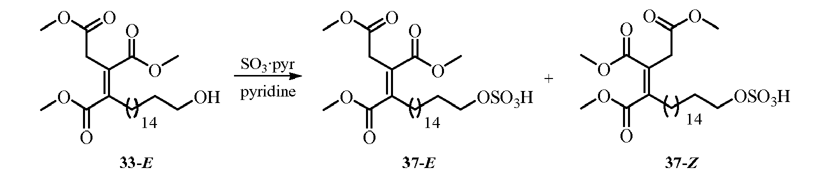

SO₃·pyr (0.181 g, 1.11 mmoles) was added in small portions to a solution of methyl (*E*)-20-hydroxy-3,4-bis(methoxycarbonyl)-3-eicosenoate **(33*-E*)** (0.102 g, 0.22 mmoles) in pyridine (4.2 ml). The mixture was stirred at room temperature for 2 hours. After that time, the solvent was removed under reduced pressure. The residue was suspended in a 1:1 H₂O/MeOH mixture (2 ml), was neutralized with an aqueous solution of 0.5M KOH and the solvent was removed under reduced pressure. The product was purified by trituration with MeOH, obtaining (0.119 g, quantitative yield) a mixture of methyl *(E*)-3,4-bis(methoxycarbonyl)-20-sulfooxy-3-eicosenoate **(37*-E*)** and methyl (*Z*)-3,4-bis(methoxycarbonyl)-20-sulfooxy-3-eicosenoate **(37*-Z*)** in a 2:3 ratio, respectively, as a white solid.
**¹H-NMR** (300 MHz, CDCl₃). δ 4.10 (2H, m, H-20 **37*-Z*),** 3.97 (2H, m, H-20 **37*-E*),** 3.79 (3H, s, -OCH₃ **37*-Z*),** 3.78 (6H, s, -OCH₃ **37**-*E*), 3.73 (3H, s, -OCH₃ **37*-Z*),** 3.69 (3H, s, -OCH₃ **37*-Z*),** 3.68 (3H, s, -OCH₃ **37*-E*),** 3.50 (2H, s, H-2 **37*-E*),** 3.41 (2H, s, H-2 **37*-Z*),** 2.60 (4H, m, -CH₂- **37**-*E*), 2.33 (4H, m, -CH₂- **37*-Z*),** 1.60-1.24 (26H, m, -CH₂-**37*-Z*, 37*-E*).**
**LRMS(EI):** *m*/*z* 536(M⁺, 0), 518(13), 486(6), 458(10), 426(5), 346(7), 287(6), 198(44), 166(88), 55(100).

### Example 19. Preparation of (Z)-3,4-dicarboxy-20-sulfooxy-3-eicosenoic acid (1-Z)

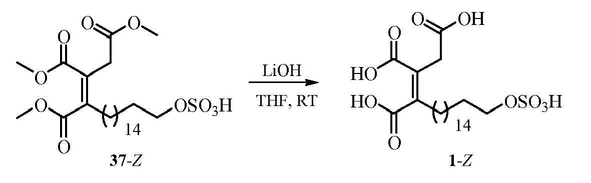

An aqueous solution of 1 M LiOH (3.3 ml) to a solution of methyl (*Z*)-3,4-bis(methoxycarbonyl)-20-sulfooxy-3-eicosenoate **(37*-Z*)** (0.059 g, 0.11 mmoles) in THF (4.7 ml) was added. The mixture was stirred at room temperature for 1 hour. After that time, H₂O (5 ml) was added. The phases were separated. The aqueous phase was washed with AcOEt (2 × 5 ml), acidified with an aqueous solution of 10% HCl and extracted with AcOEt (3 × 5 ml). The extraction organic phase was dried with anhydrous Na₂SO₄, filtered and the solvent was removed under reduced pressure, obtaining (0.048 g, yield 88%) (Z)-3,4-dicarboxy-20-sulfooxy-3-eicosenoic acid **(1*-Z*),** as a white solid. **m.p.:** 63-65 °C.
**IR (NaCl):** ν 3436, 2919, 2849, 1771, 1732, 1644, 1464, 1190, 1052 cm⁻¹.
**¹H-NMR** (300MHz, CD₃OD). δ 3.88 (2H, t, *J*= 6.5 Hz, H-20), 3.61 (2H, s, H-2), 2.23 (2H, m, -CH₂-), 1.55-1.18 (28H, m, -CH₂-).

### Example 20: Preparation of methyl (Z)-2-hydroxy-4-oxo-2-tridecenoate (84)

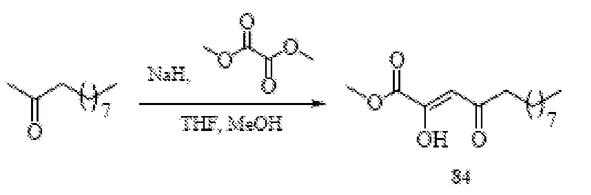

MeOH (0.5 ml) was added to a suspension ofNaH (0.253 g, 10.57 mmoles) in THF (5 ml) at 0°C. The mixture was stirred until reaching room temperature. Then, dimethyl oxalate (1.14 g, 9.69 mmoles) and a solution of 2-undecanone (1.5 g, 8.8 mmoles) in THF (3 ml) were added.32 The resulting mixture was heated at 60°C for 5 hours. After that time, AcOEt (10 ml) and an aqueous solution of 10% HCl (10 ml) were added. The phases were separated, and the aqueous phase was extracted with AcOEt (3 × 10 ml). The organic phase was dried with anhydrous Na₂SO₄, filtered and the solvent was removed under reduced pressure. The product was purified by a chromatographic column (hexane/AcOEt, 100:1), obtaining (1.18 g, yield 52%) methyl (Z)-2-hydroxy-4-oxo-2-tridecenoate **(84),** as a colorless oil.
**IR** (NaCl): ν 2922, 2853, 1739, 1720, 1648, 1543, 1437, 1018 cm⁻¹.
**¹H-NMR** (300 MHz, CDCl₃). δ 6.35 (1H, s, H-3), 3.88 (3H, s, -OCH₃), 2.46 (2H, t, *J*= 7.1 Hz, H-5), 1.63 (2H, m, H-6), 1.25 (12H, broad s, -CH₂-), 0.86 (3H, m, -CH₃). **¹³C-NMR** (75 MHz, CDCl₃). δ 203.3, 166.3, 162.6, 101.6, 53.0, 48.8, 31.7, 29.3, 29.1, 29.0, 24.8, 23.3, 20.9, 14.0.
**LRMS(EI):** *m*/*z* 256(M⁺, 0), 197(100), 168(1), 155(12), 144(12).
**E.A.** (Cl₄H₂₄O₄): Found: C, 65.50, H, 9.50; Calculated: C, 65.60, H, 9.44.

### Example 21: Preparation of methyl (2Z,4Z)-3,4-bis(methoxycarbonyl)-6-oxo-2,4-pentadecadienoate (86)

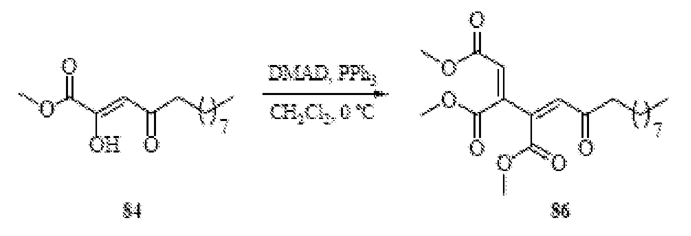

A solution of DMAD (0.138 g, 0.97 mmoles) in CH₂Cl₂ (4 ml) was added to a solution of methyl (Z)-2-hydroxy-4-oxo-2-tridecenoate **(84)** (0.250 g, 0.97 mmoles) and PPh₃ (0.255 g, 0.97 mmoles) in CH₂Cl₂ (8 ml) at 0°C. The mixture was stirred at room temperature for 24 hours. After that time, the solvent was removed under reduced pressure. The crude reaction product was dissolved in toluene (10 ml), and the mixture was heated under reflux for 6 hours. Then, the solvent was removed under reduced pressure. The product was purified by a chromatographic column (hexane/AcOEt, 6:1), obtaining (0.240 g, yield 64%) methyl (2*Z*,4*Z*)-3,4-bis(methoxycarbonyl)-6-oxo-2,4-pentadecadienoate **(86),** as a colorless oil.
**IR** (NaCl): ν 3428, 2952, 2928, 2851, 1727, 1695, 1436, 1254, 1029 cm⁻¹.
**¹H-NMR** (300 MHz, CDCl₃). δ 6.32 (1H, s, H-5), 6.11 (1H, s, H-2), 3.91 (3H, s, - OCH₃), 3.88 (3H, s, -OCH₃), 3.75 (3H, s, -OCH₃), 2.54 (2H, t, *J*= 7.3 Hz, H-7), 1.62 (2H, m, H-8), 1.24 (12H, broad s, -CH₂-), 0.86 (3H, t, *J* = 6.1 Hz, -CH₃).
**¹³C-NMR** (75 MHz, CDCl₃). δ 198.3, 166.2, 165.9, 164.4, 142.8, 138.9, 129.9, 124.5, 53.1, 53.0, 52.4, 38.6, 31.8, 29.3, 29.2, 29.0, 28.8, 23.5, 22.6, 14.0.
**LRMS(EI):** *m*/*z* 382(M⁺, 1), 351(4), 323(90), 291(11), 265(3), 255(10), 227(32), 196(100).
**E.A.** (C₂₀H₃₀O₇): Found: C, 62.90, H, 8.00; Calculated: C, 62.81, H, 7.91.

### Example 22: Reaction of methyl (2Z,4Z)-3,4-bis(methoxycarbonyl)-6-oxo-2,4-pentadecadienoate (86) with NaBH₄

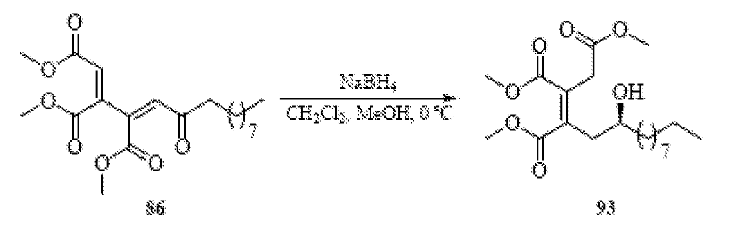

NaBH₄ (0.009 g, 0.244 mmoles) was added to a solution of methyl (2*Z*,4*Z*)-3,4-bis(methoxycarbonyl)-6-oxo-2,4-pentadecadienoate **(86)** (0.360 g, 0.941 mmoles) in a 5:2 CH₂Cl₂/MeOH mixture (21 ml) at 0°C. The mixture was stirred at 0°C for 1 hour. After that time, sat. NH₄Cl (10 ml) was added. The phases were separated, and the aqueous phase was extracted with CH₂Cl₂ (2 × 10 ml). The organic phase was dried with anhydrous Na₂SO₄, filtered and the solvent was removed under reduced pressure. The product was purified by a chromatographic column (hexane/AcOEt, 4:1), obtaining (0.020 g, yield 6%) methyl *rac*-(*Z,R*)-3,4-bis(methoxycarbonyl)-6-hydroxy-3-pentadecenoate **(93),** as a colorless oil.
**¹H-NMR** (300 MHz, CDCl₃). δ 3.80 (3H, s, -OCH₃), 3.74 (3H, s, -OCH₃), 3.70 (3H, s, - OCH₃), 3.66 (1H, m, H-6), 3.46 (2H, s, H-2), 2.48 (2H, d, *J*= 6.0 Hz, H- 5), 1.64-1.43 (4H, m, -CH₂-), 1.25 (16H, broad s, -CH₂-), 0.86 (3H, m, -CH₃).
**LRMS(EI):** *m*/*z* 386(M+, 0), 353(0), 320(2), 295(1), 265(0), 235(1), 198(91), 166(100), 139(28),111(10).

## Claims

1. A process for obtaining a compound of formula (VIII), its stereoisomers or mixtures thereof 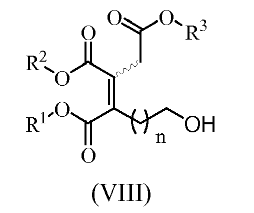 wherein
R¹, R² and R³ are independently selected from substituted or unsubstituted C₁-C₂₀ alkyl; and
n is an integer between 2 and 20;
**characterized in that** it comprises at least one of the following steps (a), (b) or (c)
(a) subjecting a compound of formula (VI), its stereoisomers or mixtures thereof, to a hydrogenation reaction 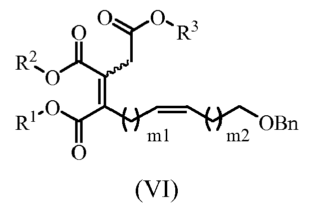 wherein
R¹, R² and R³ are as defined above;
m1 is an integer which is selected from 1, 2, 3, 4 and 5; and
m2 is an integer between 0 and 13;
(b) subjecting a compound of formula (VII), its stereoisomers or mixtures thereof, to a hydrogenation reaction 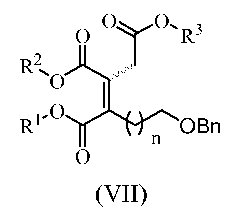 wherein
n, R¹, R² and R³ are as defined above;
or
(c) removing the trialkylsilyl group of a compound of formula (XVI), its stereoisomers or mixtures thereof 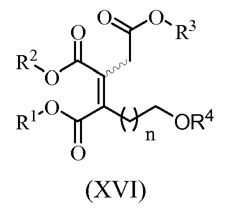 wherein
n, R¹, R² and R³ are as defined above; and
R⁴ is a trialkylsilyl group.

2. The process according to claim 1, wherein said hydrogenation uses Pd/C as a catalyst and 1,4-dioxane as a solvent.

3. The process according to any of claims 1 or 2, wherein the compound of formula (VIII), its stereoisomers or mixtures thereof, is a compound of formula (VIIIa), its stereoisomers or mixtures thereof, 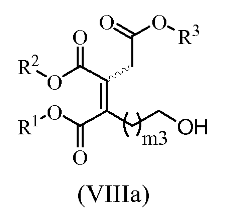 wherein
R¹, R² and R³ are independently selected from substituted or unsubstituted C₁-C₂₀ alkyl; and
m3 is an integer between 2 and 18;
which additionally comprises the following steps
(i) activating as a leaving group the hydroxyl group of a compound of formula (VIIIa), its stereoisomers or mixtures thereof, to form a compound of formula (XVIII), its stereoisomers or mixtures thereof 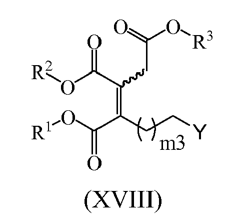 wherein
m3, R¹, R² and R³ are as defined above; and
Y is selected from -OSO₃H, -OMs, -OTs, I and Br;
(ii) reacting said compound of formula (XVIII), its stereoisomers or mixtures thereof, with a compound of formula (X) 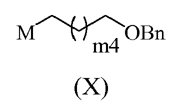 wherein
M is a metal, preferably selected from among Grignard reagents transmetalated or non-transmetalated with Zn, Al, or Cu; and
m4 is an integer between 0 and 18; and
the relation m3 + m4 + 2= n is met;
to obtain a compound of formula (VII), its stereoisomers or mixtures thereof 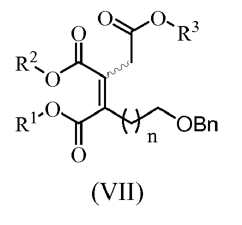 wherein
n, R¹, R² and R³ are as defined above,
and
(iii) subjecting said compound of formula (VII), its stereoisomers or mixtures thereof, to a hydrogenation reaction.

4. A compound of formula (VII) 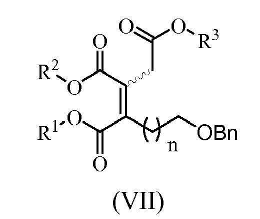 wherein
n, R¹, R² and R³ are as defined in claim 1;
its stereoisomers or mixtures thereof.

5. A process for the synthesis of a compound of formula (VII) as defined in claim 4, its stereoisomers or mixtures thereof, **characterized in that** it comprises the selective hydrogenation of the double bond of the compound of formula (VI), as defined in claim 1, its stereoisomers or mixtures thereof.

6. A compound of formula (VI) 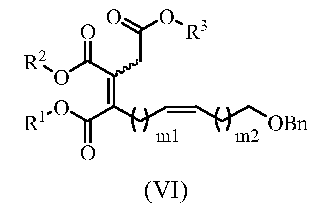 wherein
m1, m2, R¹, R² and R³ are as defined in claim 1;
its stereoisomers or mixtures thereof.

7. A process for obtaining a compound of formula (VI) as defined in claim 6, its stereoisomers or mixtures thereof, **characterized in that** it comprises the following steps
(i) reacting a compound of formula (III) with a compound of formula (XI), to yield a compound of formula (IV) 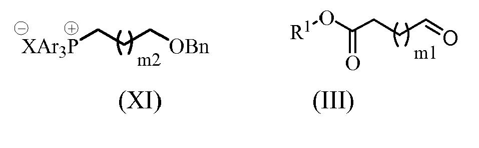 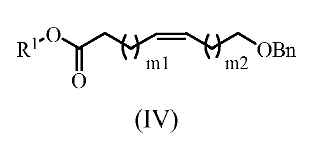 wherein
R¹ is selected from a substituted or unsubstituted C₁-C₂₀ alkyl,
m1 is an integer which is selected from 1, 2, 3, 4 and 5;
m2 is an integer between 0 and 13;
X is a halogen; and
each of the Ar groups is independently selected from among C₆-C₁₀ aryl groups.
(ii) reacting said compound of formula (IV) with an oxalate of formula (XII) in the presence of a base to yield a compound of formula (V) 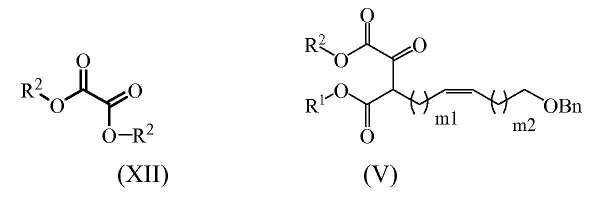 wherein
m1, m2, R¹ and R² are as defined above;
and
(iii) reacting said compound of formula (V) with a phosphonate of formula (XIII) 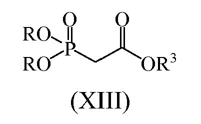
wherein
R¹ is selected from a substituted or unsubstituted C₁-C₂₀ alkyl; and
R is selected from C₁-C₄ alkyl substituted or unsubstituted with one or more halogen atoms, benzyl, phenyl and tolyl.

8. The process according to claim 7, **characterized in that** the preparation of said compound of formula (III) comprises the steps of
(i) reacting a compound of formula (XXIVa) in the presence of an alcohol of formula R¹OH to yield a compound of formula (IIa) 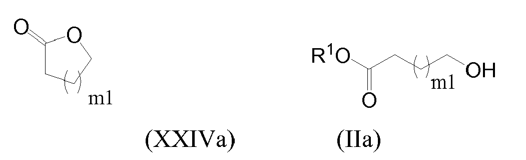 wherein
R¹ is selected from a substituted or unsubstituted C₁-C₂₀ alkyl; and
m1 is an integer which is selected from 0, 1, 2, 3, 4 and 5;
and
(ii) oxidizing the primary hydroxyl of the compound of formula (IIa) to aldehyde.

9. A compound of formula (XVI), its stereoisomers or mixtures thereof 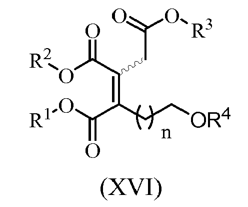 wherein
n is an integer between 2 and 20;
R¹, R² and R³ are independently selected from a substituted or unsubstituted C₁-C₂₀ alkyl; and
R⁴ is a trialkylsilyl group;

10. A process for the synthesis of a compound of formula (XVI) as defined in claim 9, its stereoisomers or mixtures thereof, **characterized in that** it comprises the steps of
(i) reacting a compound of formula (XXIV) in the presence of an alcohol of formula R¹OH to yield a compound of formula (II) 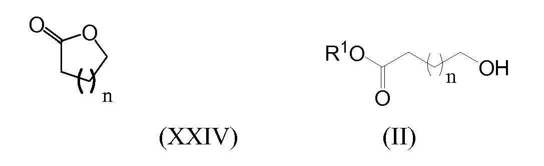 wherein
R¹ is selected from a substituted or unsubstituted C₁-C₂₀ alkyl; and
n is an integer between 2 and 20;
(ii) protecting the free hydroxyl group of said compound of formula (II) with a trialkylsilyl to obtain a compound of formula (XIV) 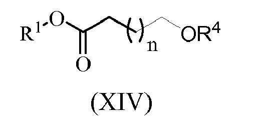 wherein
R¹ and n are as defined above; and
R⁴ is a trialkylsilyl group;
(iii) reacting said compound of formula (XIV) with an oxalate of formula (XII) in the presence of a base to obtain a compound of formula (XV) 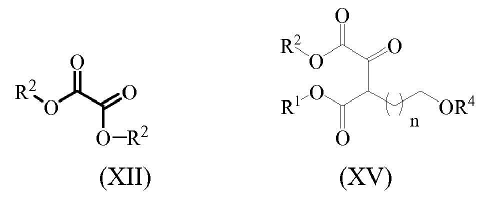 wherein
R² is selected from a substituted or unsubstituted C₁-C₂₀ alkyl; and
n, R¹ and R⁴ are as defined above;
and
(iv) reacting said compound of formula (XV) with a phosphonate of formula (XIII) in the presence of a base 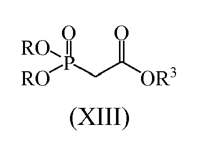 wherein
R is selected from a substituted or unsubstituted C₁-C₄ alkyl with one or more halogen atoms, benzyl, phenyl and tolyl; and
R³ is selected from a substituted or unsubstituted C₁-C₂₀ alkyl.

11. A process for the synthesis of a compound of formula (I), 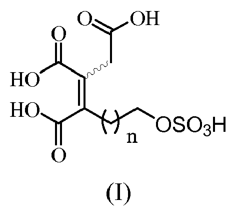 wherein n is an integer between 2 and 20;
its stereoisomers or mixtures thereof, **characterized in that** it comprises at least one of the following steps (ai), (aii) or (aiii)
(ai) subjecting a compound of formula (VI), its stereoisomers or mixtures thereof, to a hydro genation 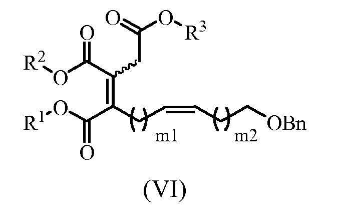 or
(aii) subjecting a compound of formula (VII), its stereoisomers or mixtures thereof, to a hydro genation 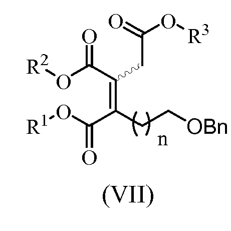 or
(aiii) removing the trialkylsilyl group of a compound of formula (XVI), its stereoisomers or mixtures thereof 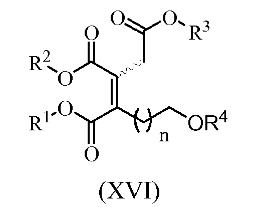 wherein
n is an integer between 2 and 20;
R¹, R² and R³ are independently selected from a substituted or unsubstituted C₁-C₂₀ alkyl;
m1 is an integer which is selected from 0, 1, 2, 3, 4 and 5;
m2 is an integer between 0 and 13; and
R⁴ is a trialkylsilyl group;
to obtain a compound of formula (VIII), its stereoisomers or mixtures thereof 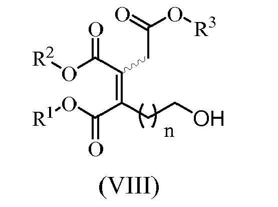 wherein
n, R¹, R² and R³ are as defined above;
and additionally comprises
(b) reacting said compound of formula (VIII), its stereoisomers or mixtures thereof, with a sulfating agent to yield a compound of formula (IX), its stereoisomers or mixtures thereof 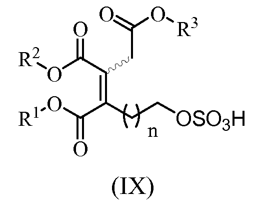 wherein
n, R¹, R² and R³ are as defined above;
and
(c) hydrolyzing the ester groups of said compound of formula (IX), its stereoisomers or mixtures thereof, to provide the corresponding acid groups.

12. The process according to any of the previous claims, which comprises obtaining a compound of formula cis-(I), cis-(VI), cis-(VII), cis-(VIII), cis-(VIIIa), cis-(IX), cis-(XVI), cis-(XVIa) or cis-(XVIII) or the enrichment in the cis-isomer of a mixture of cis- and trans-isomers of a compound of formula (I), (VI), (VII), (VIII), (VIIIa), (IX), (XVI), (XVIa) or (XVIII), by means of the exposure to a basic medium of a compound of formula trans-(I), trans-(VI), trans-(VII), trans-(VIII), trans-(VIIIa), trans-(IX), trans-(XVI), trans-(XVIa) or trans-(XVIII), or a mixture of cis- and trans-isomers of a compound of formula (I), (VI), (VII), (VIII), (VIIIa), (IX), (XVI), (XVIa) or (XVIII).

13. A compound of formula (VIII), its stereoisomers or mixtures thereof 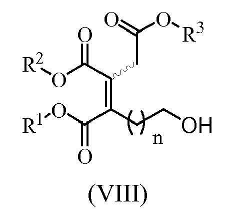 wherein
R¹, R² and R³ are independently selected from substituted or unsubstituted C₁-C₂₀ alkyl; and
n is an integer between 0 and 20;
with the exception of a compound of formula

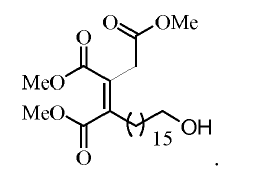

14. A compound of formula (I), its stereoisomers or mixtures thereof, 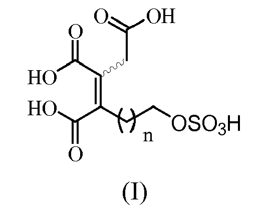 wherein n is an integer between 2 and 20;
except

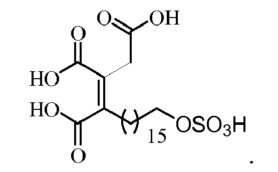

15. A compound of formula (IX), its stereoisomers or mixtures thereof 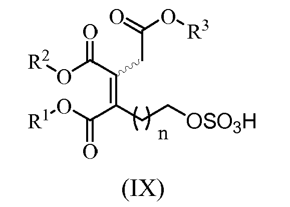 wherein
n is an integer between 2 and 20; and
R¹, R² and R³ are independently selected from a substituted or unsubstituted C₁-C₂₀ alkyl;
except

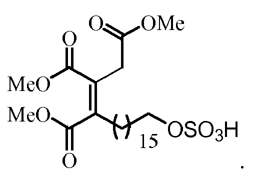

16. A compound of formula (IV), its stereoisomers or mixtures thereof 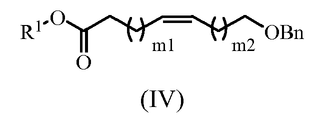 wherein
R¹ is selected from a substituted or unsubstituted C₁-C₂₀ alkyl,
m1 is an integer which is selected from 0, 1, 2, 3, 4 and 5;
m2 is an integer between 0 and 13.

17. A compound of formula (V), its stereoisomers or mixtures thereof 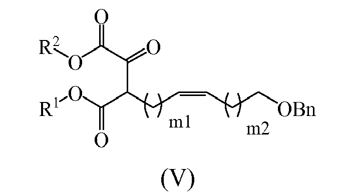 wherein
R¹ and R² are selected from a substituted or unsubstituted C₁-C₂₀ alkyl, m1 is an integer which is selected from 0, 1, 2, 3, 4 and 5;
m2 is an integer between 0 and 13.

18. A compound of formula (XV), its stereoisomers or mixtures thereof 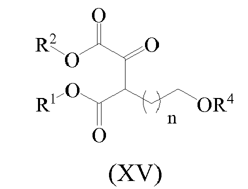 wherein
n is an integer between 2 and 20;
R¹ and R² are independently selected from a substituted or unsubstituted C₁-C₂₀ alkyl; and
R⁴ is a trialkylsilyl group.

19. A compound of formula (XVIII), its stereoisomers or mixtures thereof 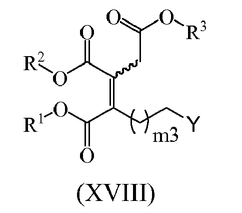 wherein
R¹, R² and R³ are independently selected from a substituted or unsubstituted C₁-C₂₀ alkyl;
m3 is an integer between 2 and 18; and
Y is selected from -OSO₃H, OTs and Br.

20. Use of at least one compound selected from the compounds of formula (II), (III), (IV), (V), (VI), (VII), (VIII), (X), (XI), (XII), (XIII), (XIV), (XV), (XVI), (XVIII) and (XXIV) as defined in the previous claims, their stereoisomers or mixtures thereof, for the synthesis of a compound of formula (I), its stereoisomers or mixtures thereof.
